(19) Europäisches Patentamt
European Patent Office
Office européen des brevets

(11) **EP 3 985 090 A1**

(12) **EUROPEAN PATENT APPLICATION**
published in accordance with Art. 153(4) EPC

(43) Date of publication:
**20.04.2022 Bulletin 2022/16**

(21) Application number: **20822005.3**

(22) Date of filing: **03.06.2020**

(51) International Patent Classification (IPC):
**C12M 1/00** (2006.01)    **A01N 1/02** (2006.01)
**C12N 1/04** (2006.01)    **C12N 5/073** (2010.01)

(52) Cooperative Patent Classification (CPC):
**A01N 1/02; C12M 1/00; C12N 1/04**

(86) International application number:
**PCT/JP2020/021895**

(87) International publication number:
**WO 2020/250766 (17.12.2020 Gazette 2020/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.06.2019   JP 2019109751
21.10.2019   JP 2019191857
28.01.2020   JP 2020011785
14.05.2020   JP 2020084840**

(71) Applicant: **Mitsubishi Paper Mills Limited
Tokyo 130-0026 (JP)**

(72) Inventors:
• **MATSUZAWA, Atsushi
Tokyo 130-0026 (JP)**
• **YOSHIDA, Kakeru
Tokyo 130-0026 (JP)**

(74) Representative: **Cabinet Beau de Loménie
158, rue de l'Université
75340 Paris Cedex 07 (FR)**

(54) **FIXTURE OF CRYOPRESERVATION JIG AND FREEZING AND THAWING METHOD USING SAID FIXTURE**

(57)    Provided is a cryopreservation jig fixture including: a base having an upper surface, side surfaces, and a bottom surface; and either a slit on the upper surface of the base or an insertion portion for inserting a cryopreservation jig on the upper surface of the base and a slit having one end opened at a lateral portion of the insertion portion. Also provided is a method of freezing and thawing a cell or tissue, which uses the fixture.

FIG.33

**Description**

TECHNICAL FIELD

The present invention relates to a cryopreservation jig fixture suitable for use in thawing of frozen cells or tissues, and a freezing and thawing method that uses the fixture.

BACKGROUND ART

Excellent cell or tissue preservation techniques are desired in various industrial fields. For example, in the bovine embryo transfer technology, embryos are cryopreserved in advance and thawed and transferred according to the estrous cycle of a recipient cow. In the human fertility treatment, eggs or ovaries harvested from a woman's body are cryopreserved until appropriate timing for transplantation, and the cryopreserved eggs or ovaries are thawed for use in transplantation.

Generally, cells or tissues harvested from living bodies gradually become inactive and/or undergo transformation even in a culture medium. Thus, long-term culture of cells or tissues in vitro is undesirable. For this reason, techniques for long-term preservation of cells or tissues with their bioactivity maintained are essential. Excellent preservation techniques enable more accurate analysis of cells or tissues harvested. Such excellent preservation techniques also enable transplantation of cells or tissues with their bioactivity maintained at a higher level, thus likely improving the engraftment rate. The techniques also enable sequential production and preservation of artificial tissues for transplantation, such as skins cultured in vitro and what is called "cell sheets" formed in vitro, and storage thereof until needed. Therefore, such excellent preservation techniques are expected to bring great advantages not only in the medical science fields but also in the industrial fields.

One of known cell or tissue cryopreservation methods is a slow freezing method, for example. In this method, cells or tissues are immersed in a preservation solution prepared by adding a cryoprotectant to a physiological solution such as phosphate buffered saline, for example. Examples of the cryoprotectant include compounds such as glycerol, ethylene glycol, and dimethyl sulfoxide. The cells or tissues immersed in the preservation solution are cooled down to -30°C to -35°C at a relatively slow cooling rate (for example, 0.3°C to 0.5°C/min), whereby the solution inside and outside the cells or tissues is sufficiently cooled and increases its viscosity. Further cooling down the cells or tissues in the preservation solution in such a state to the temperature of liquid nitrogen (-196°C) allows a slight amount of the solution both inside and outside (surrounding) the cells or tissues to become a solid while the amorphous state thereof is maintained, that is, to vitrify. Vitrification solidifies the solution inside and outside the cells or tissues, which substantially immobilizes the molecules. Thus, the vitrified cells or tissues can be semipermanently preserved in the liquid nitrogen.

Another known cell or tissue cryopreservation method is a vitrification method. The vitrification method is a technique using a principle that addition of a large amount of a cryoprotectant, such as glycerol, ethylene glycol, or dimethyl sulfoxide (DMSO), to a preservation solution decreases the freezing point of the preservation solution, which reduces or prevents ice crystal formation at sub-zero temperatures. When quickly cooled in liquid nitrogen, the preservation solution can solidify without forming ice crystals. This solidification is called vitrification. The preservation solution containing a large amount of a cryoprotectant is called a vitrification solution.

The slow freezing method described above requires cooling at a relatively slow cooling rate, which prolongs the operation of cryopreservation. Disadvantageously, this method also requires a device or jig for controlling the cooling rate. In addition, the slow freezing method cannot avoid ice crystal formation in the preservation solution outside the cells or tissues, which may cause physical damage to the cells or tissues. In contrast, the vitrification method described above is a process that shortens the operation time and requires no special devices or jigs. In addition, the vitrification method provides high viability because it prevents ice crystal formation.

There have been reported various examples of cell or tissue cryopreservation by the vitrification method using various methods and various cells or tissues. For example, according to Patent Literature 1, application of the vitrification method to animal or human reproductive or somatic cells is very useful in terms of viability after cryopreservation and thawing.

The vitrification method is a technique which has been developed mainly using human reproductive cells. More recently, its application to iPS or ES cells has also been widely examined. Non-Patent Literature 1 discloses the effectiveness of the vitrification method in preservation of Drosophila embryos. Patent Literature 2 discloses the effectiveness of the vitrification method in preservation of plant culture cells and tissues. As mentioned here, the vitrification method is known to be useful for preservation of various kinds of cells and tissues.

It is known that a higher freezing rate is better for suitable vitrification. Also in a thawing step after cryopreservation, it is known that a higher freezing rate is better for reduction or prevention of ice crystal reformation in cells or tissues.

The freezing rate and the thawing rate are important factors for suitable vitrification. Of these, the thawing rate is considered to be particularly important. For example, as described in Non-Patent Literature 2, it is known that rapidly frozen cells may have a low viability, if the thawing rate is slow. According to Patent Literature 3, the viability of human induced pluripotent stem (iPS) cell-derived neurons/precursor cells after thawing is improved by thawing frozen samples by the rapid thawing method.

As a general freezing method applicable to the vitrification method, Patent Literature 4 suggests a method in which mammalian embryos or eggs are attached to an inner surface of a cryopreservation container such as a cryostraw, cryovial, or cryotube, with a vitrification solution in a minimum amount sufficient to cover these embryos or eggs, and the container is brought into contact with liquid nitrogen for rapid cooling. In a thawing method that is performed after the freezing method, the cryopreservation container stored by the above-described method is taken out from the liquid nitrogen, and one end of the container is opened to inject a diluent at 33°C to 39°C into the container, whereby the frozen embryos or eggs are thawed and diluted. This method is considered to be capable of preserving, thawing, and diluting mammalian embryos or eggs with high viability, without risk of infection with viruses and/or bacteria. However, it is highly difficult to attach embryos or eggs to an inner surface of a cryopreservation container such as a cryostraw, cryovial, or cryotube, and it is also difficult to confirm that the embryos or eggs are deposited in the cryopreservation container.

Patent Literature 5 discloses a cell cryopreservation tool including a cell holding member having a thermal conductive member and a tubular storage member. With this tool, the problems in the freezing and thawing method disclosed in Patent Literature 4 are solved to some degree. The cryopreservation tool disclosed in Patent Literature 5 is used in the following manner: eggs are attached to the cell holding member under a microscope; the cell holding member is housed in the tubular storage member; and the cryopreservation tool is then immersed in liquid nitrogen for vitrification. Subsequently, according to a storage method disclosed in Patent Literature 5, a lid is attached to an opening of the tubular storage member, and the tubular storage member is stored in a liquid nitrogen tank. According to Patent Literature 6, a cryopreservation jig including an egg-holding strip on which eggs are deposited with a small amount of a vitrification solution is entirely housed in a tubular storage container, and then immersed in liquid nitrogen for vitrification.

Patent Literature 7 and Patent Literature 8 each disclose a freezing method applicable to the vitrification method with fewer steps, which is called the Cryotop® method that has been used in the field of human fertility treatment. Freezing in these methods is performed using an egg cryopreservation tool including a flexible, clear, and colorless film strip as an egg-holding strip. Eggs or embryos are deposited with a very small amount of a preservation solution on the film under microscope observation, and the film with the eggs attached thereto is immersed in liquid nitrogen for vitrification. The egg-holding strip holding the frozen eggs or embryos is protected by a cap or the like, and then stored in a liquid nitrogen tank.

As a cryopreservation jig suitable for the Cryotop method, for example, Patent Literatures 9 to 11 each suggest a method of cryopreserving eggs or embryos with high viability by using a cryopreservation jig with absorbency to remove an excess preservation solution surrounding these reproductive cells.

Eggs or embryos frozen by these methods are thawed by immersing the egg-holding strip in a thawing solution kept warm, and the eggs or embryos deposited on the strip are recovered in the thawing solution.

Meanwhile, for example, Patent Literature 12 discloses a liquid nitrogen container intended to improve working efficiency in thawing in the cryopreservation method. The liquid nitrogen container includes a lid having a slit formed therein. A cryopreservation jig housed in a tubular storage member is temporarily leaned against the slit.

In this manner, the cryopreservation jig is temporarily held in a state where the cells or tissues frozen are below a liquid surface of the liquid nitrogen. Thus, for example, in the methods disclosed in Patent Literature 5 and Patent Literature 6, the lid above the liquid surface of the liquid nitrogen can be easily removed, which allows the cells or tissues to be rapidly transferred to the thawing solution, thus preventing the slowdown of the thawing rate, while in the methods disclosed in Patent Literatures 7, 8, and 9 to 11, the cells or tissues can be rapidly transferred to the thawing solution by pulling out the cryopreservation jig from the cap, thus preventing the slowdown of the thawing rate.

CITATION LIST

- Patent Literature

Patent Literature 1: JP 3044323 B
Patent Literature 2: JP 2008-5846 A
Patent Literature 3: JP 2017-104061 A
Patent Literature 4: JP 2000-189155 A
Patent Literature 5: JP 5798633 B
Patent Literature 6: WO 2019/004300
Patent Literature 7: JP 2002-315573 A
Patent Literature 8: JP 2006-271395 A

Patent Literature 9: JP 2014-183757 A
Patent Literature 10: JP 2015-142523 A
Patent Literature 11: WO 2015/064380
Patent Literature 12: U.S. Design Patent No. D642,697

- Non-Patent Literature

Non-Patent Literature 1: Steponkus et al., Nature 345:170-172 (1990)
Non-Patent Literature 2: "Seisaibo no toketsu ni yoru shogai to hogo no kiko" (Mechanism of damage to living cells by freezing and protection) written by Hiroshi Souzu, Kagaku to Seibutsu (Chemistry and Biology), vol. 18 (1980), no. 2. pp. 78-87, published by The Japan Society for Bioscience, Biotechnology, and Agrochemistry

SUMMARY OF INVENTION

- Technical problem

However, in the method that uses the liquid nitrogen container according to Patent Literature 12, while it is possible to stably hold a cryopreservation jig when it is leaned against the slit formed in the lid, it is difficult to confirm that a deposition part of the cryopreservation jig is below the liquid surface of the liquid nitrogen and the frozen state of the cells or tissues is maintained. In addition, the length of the tubular storage member is required to be longer than the length from the bottom of the liquid nitrogen container to the lid. In such a case, taking out the body of the cell holding member from the inside of the tubular resin member is complicated, and improvement has been required in terms of rapid thawing.

There have been suggested methods of cryopreserving eggs or embryos with a small amount of a preservation solution to provide high viability, for example, by limiting the width of the film on which eggs or embryos are to be deposited (Patent Literatures 7 and 8), or by using the cryopreservation jig with absorbency to remove an excess preservation solution surrounding eggs or embryos (Patent Literatures 9 to 11). However, in these methods, air bubbles are attached to the film during thawing in which the embryos or eggs are thawed and recovered, making it difficult to distinguish the deposited embryos or eggs from the air bubbles under microscope observation. Further, the air bubbles attached to the film may be attached to the eggs or embryos, impairing their visibility, or the eggs or embryos may suddenly disappear from the microscopic field due to buoyancy of the air bubbles. These problems interfere with the recovery of the embryos or eggs.

A main object of the present invention is to provide a fixture for achieving good working efficiency in thawing in cell or tissue vitrification. More specifically, the present invention aims to provide a cryopreservation jig fixture that can stably hold a cryopreservation jig; that allows, prior to transfer of frozen cells or tissues to a thawing solution, an operator to confirm that the cells or tissues are in a cooled state; and that enables rapid transfer of the frozen cells or tissues to the thawing solution kept warm.

The present invention also aims to provide a freezing and thawing method that facilitates recovery of cells or tissues when thawing the cells or tissues after freezing, without requiring complicated work.

As a result of extensive studies to solve the above problems, the present inventors found that a cryopreservation jig fixture configured as described below (hereinafter also referred to as "the fixture of the present invention") can solve the above problems. The present inventors also found that a method of freezing and thawing a cell or tissue described below can solve the above problems.

(1) A cryopreservation jig fixture including: a base having an upper surface, side surfaces, and a bottom surface; and either a slit on the upper surface of the base having at least one end opened at the side surface of the base or an insertion portion for inserting a cryopreservation jig on the upper surface of the base and a slit having one end opened at a lateral portion of the insertion portion, wherein the slit has a fixing structure for fixing a cryopreservation jig, and the fixing structure is at least one of the following structures (I) to (III):

(I) an irregular structure formed on at least one inner side surface of the slit;
(II) a tapered structure in which a distance between inner side surfaces of the slit gradually decreases toward a back of the slit; and
(III) a bent structure in which the slit extends linearly from a slit opening toward an inside of the base and then bends in a direction different from the linearly extending direction.

(2) The cryopreservation jig fixture according to (1) above, wherein the slit has at least one of the tapered structure (II) and the bent structure (III) as the fixing structure.

(3) The cryopreservation jig fixture according to (1) or (2) above, wherein the slit has the tapered structure (II) as the fixing structure, and the tapered structure satisfies the following formula (1):

$$(T21 - T22)/T11 \leq 0.03 \quad \text{formula (1)}$$

where T21 is a width of the tapered structure at a portion closest to the slit opening, T22 is a width of the tapered structure at a portion farthest from the slit opening, and T11 is a length of the tapered structure.

(4) The cryopreservation jig fixture according to any one of (1) to (3) above, wherein the slit has, as the fixing structure, the bent structure (III) in which the slit bends in a direction different from its linearly extending direction and the tapered structure (II) located after the bend.

(5) The cryopreservation jig fixture according to any one of (1) to (4) above, further including a liquid surface gauge portion on the upper surface or one of the side surfaces of the base, the liquid surface gauge portion extending above the upper surface of the base.

(6) A method of freezing and thawing a cell or tissue, including the following steps: a sealing step of depositing the cell or tissue with a preservation solution on a deposition part of a cryopreservation jig at least including a body including the deposition part and a cap member freely attachable to and detachable from the body, and sealing the deposition part with the cap member; a freezing step of cooling the sealed deposition part by a coolant to freeze the cell or tissue; a refrigerating step of maintaining the frozen state of the cell or tissue frozen in the freezing step; and a thawing step of thawing the cell or tissue maintained in the frozen state, wherein prior to the thawing step, the cell or tissue sealed with the cap member is fixed to the fixing structure of the slit of the cryopreservation jig fixture according to any one of (1) to (5) above, and the cryopreservation jig is temporarily held in a state where the cell or tissue is below a liquid surface of the coolant and a joint portion between the body and the cap member is above the liquid surface of the coolant, and in the thawing step, the body and the cap member are separated from each other, and the deposition part of the body is immersed in a thawing solution to thaw the cell or tissue.

(7) The method of freezing and thawing a cell or tissue according to (6) above, wherein the deposition part includes a preservation solution absorber.

- Advantageous Effects of Invention

The present invention can provide a fixture for achieving good working efficiency in thawing in cell or tissue vitrification. More specifically, the present invention can provide a cryopreservation jig fixture that can stably hold a cryopreservation jig; that allows, prior to transfer of frozen cells or tissues to a thawing solution, an operator to confirm that the cells or tissues are in a cooled state; and that enables rapid transfer of the frozen cells or tissues to the thawing solution kept warm.

The present invention can also provide a freezing and thawing method that facilitates recovery of cells or tissues when thawing the cells or tissues after freezing, without requiring complicated work.

BRIEF DESCRIPTION OF DRAWINGS

Fig. 1 is a schematic top view of an example of a cryopreservation jig to be fixed with a fixture of the present invention.
Fig. 2 is a schematic side view of an example of the cryopreservation jig to be fixed with the fixture of the present invention.
Fig. 3 is a side cross-sectional view of an example of a cap member of the cryopreservation jig to be fixed with the fixture of the present invention.
Fig. 4 is a side view of an example of the cap member of the cryopreservation jig to be fixed with the fixture of the present invention, as viewed from an opening side.
Fig. 5 is a schematic view of an example of a body and the cap member being fitted together and fixed.
Fig. 6 is a schematic side view of a cell and a preservation solution being deposited on the cryopreservation jig.
Fig. 7 is a schematic side view of the cap member being fitted and fixed after the cell and the preservation solution are deposited on the cryopreservation jig.
Fig. 8 is a schematic top view of an example of the fixture of the present invention.
Fig. 9 is a schematic top view of another example of the fixture of the present invention.
Fig. 10 is a schematic side view of an example of a slit of the fixture of the present invention.

Fig. 11 is a schematic side view of another example of the slit of the fixture of the present invention.
Fig. 12 is a schematic side view of still another example of the slit of the fixture of the present invention.
Fig. 13 is a schematic top view of another example of the fixture of the present invention.
Fig. 14 is a schematic top view of still another example of the fixture of the present invention.
Fig. 15 is a schematic top view of still another example of the fixture of the present invention.
Fig. 16 is a schematic top view of still another example of the fixture of the present invention.
Fig. 17 is a schematic top view of still another example of the fixture of the present invention.
Fig. 18 is a schematic top view of still another example of the fixture of the present invention.
Fig. 19 is a schematic top view of still another example of the fixture of the present invention.
Fig. 20 is a schematic top view of still another example of the fixture of the present invention.
Fig. 21 is a schematic top view of still another example of the fixture of the present invention.
Fig. 22 is a schematic top view of still another example of the fixture of the present invention.
Fig. 23 is a schematic side view of still another example of the slit of the fixture of the present invention.
Fig. 24 is a schematic side view of still another example of the fixture of the present invention.
Fig. 25 is a schematic side view of still another example of the fixture of the present invention.
Fig. 26 is a schematic side view of still another example of the fixture of the present invention.
Fig. 27 is a schematic side view of still another example of the fixture of the present invention.
Fig. 28 is a schematic side view of still another example of the fixture of the present invention.
Fig. 29 is a schematic top view of still another example of the fixture of the present invention.
Fig. 30 is a schematic side view of still another example of the fixture of the present invention.
Fig. 31 is a schematic view of a cell or tissue sealed with the cap member being below a liquid surface of a coolant and a joint portion of the cap member being above the liquid surface of the coolant, prior to a thawing step.
Fig. 32 is another schematic view of the cell or tissue sealed with the cap member being below the liquid surface of the coolant and the joint portion of the cap member being above the liquid surface of the coolant, prior to the thawing step.
Fig. 33 is a schematic view of the body and the cap member being separated from each other, and a deposition part being immersed in a thawing solution.

DESCRIPTION OF EMBODIMENTS

The cryopreservation jig fixture of the present invention (hereinafter described as "the fixture of the present invention") is used to fix a cell or tissue cryopreservation jig. The fixture of the present invention is also suitably used for cell or tissue cryopreservation called "vitrification method". The term "cell" herein encompasses not only a single cell but also a biological cell population composed of multiple cells. The cell population composed of multiple cells may be a cell population composed of a single kind of cells or may be a cell population composed of multiple kinds of cells. The tissue may be composed of a single kind of cells or may be composed of multiple kinds of cells, or may contain a non-cellular substance like an extracellular matrix in addition to the cells. The fixture of the present invention can be particularly suitably used in egg or embryo cryopreservation.

The fixture of the present invention can also be referred to as a "fixture for a cell or tissue cryopreservation jig", a "fixing tool for a cell or tissue cryopreservation jig", a "fixing device for a cell or tissue cryopreservation jig", a "fixture for a cell or tissue cryopreservation tool", or a "fixture for a cell or tissue cryopreservation device".

Hereinafter, the fixture of the present invention is described in detail.

The fixture of the present invention is a cryopreservation jig fixture, which includes a base having an upper surface (top surface), side surfaces, and a bottom surface, and a slit on the upper surface of the base for fixing a cryopreservation jig. Alternatively, the fixture includes, on the upper surface of the base, an insertion portion into which a cryopreservation jig is inserted and a slit whose one end is opened at a lateral portion of the insertion portion. In the present invention, the fixture only needs one slit on the upper surface of the base, but may include multiple slits.

In fixing a cryopreservation jig using the fixture of the present invention, a tip of a tubular storage member of the cryopreservation jig or a tip of a cap member of the cryopreservation jig is inserted into the slit of the fixture of the present invention, and the cryopreservation jig is moved along the slit to the back of the slit. The width of the slit of the fixture of the present invention is preferably 90 to 110% relative to the width of the tip of the tubular storage member or the width of the tip of the cap member in order to achieve both handleability when moving the cryopreservation jig and fixing stability of the cryopreservation jig. The tubular storage member may be one having one end blocked, which is capable of housing a cell holding member and is formed from a cold-resistant material, as disclosed in Patent Literature 5.

The slit of the fixture of the present invention has a fixing structure for securely fixing and holding the cryopreservation jig inserted thereinto, and the slit has at least one of the following structures (I) to (III) as the fixing structure:

(I) an irregular structure formed on at least one inner side surface of the slit;

(II) a tapered structure in which a distance between inner side surfaces of the slit gradually decreases toward a back of the slit; and

(III) a bent structure in which the slit extends linearly from a slit opening toward an inside of the base and then bends in a direction different from the linearly extending direction.

The following describes the irregular structure formed on at least one inner side surface of the slit. The irregular structure formed on at least one inner side surface of the slit can reliably fix a cryopreservation jig to the fixture when the cryopreservation jig has an irregular structure on its tip (the tip of the tubular storage member or the tip of the cap member), because the irregular structure of the fixture and the irregular structure of the cryopreservation jig fit into each other. Preferably, the irregular structure on at least one inner side surface of the slit has a shape of a recess and/or protrusion extending in a direction parallel to the bottom surface of the fixture.

In the fixture of the present invention, when the slit includes the irregular structure on at least one inner side surface thereof, the irregular structure may be a recessed structure and/or a protruding structure of any shape according to the shape of the tip of the cryopreservation jig to be fixed, preferably, according to the shape of the tip of the cap member. Preferably, the irregular structure of the slit of the fixture has a cross-sectional shape that is the same as the cross-sectional shape of the irregular structure of the tip of the cryopreservation jig to be fixed.

In the fixture of the present invention, when the slit includes the irregular structure on at least one inner side surface thereof, it suffices as long as the slit includes at least one irregular structure on the inner side surface thereof. In order to increase the fixing stability of the cryopreservation jig, it is more preferred that the slit includes the irregular structures on both side surfaces facing each other inside the slit. Here, the expression "both side surfaces facing each other inside" refers to both side surfaces facing each other in a schematic top view when the fixture of the present invention is viewed from the top. The irregular structures on both side surfaces may be continuously connected to each other in the back of the slit.

When the slit of the fixture of the present invention has the tapered structure as the fixing structure, the slit is configured such that the distance between the inner side surfaces of the slit gradually decreases from the slit opening into which the cryopreservation jig is inserted toward the back of the slit. When the slit of the fixture of the present invention has such a tapered structure, advantageously, the cryopreservation jig inserted can be reliably fixed to the fixture because it is fitted into the tapered structure. The tapered structure may have any shape according to the shape of the tip of the cryopreservation jig to be fixed, specifically, the shape of the tip of the tubular storage member or the tip of the cap member. It is favored that the tapered structure satisfies the following formula (1), because the resulting fixture will be particularly excellent in terms of fixing stability of the cryopreservation jig.

$$(T21 - T22)/T11 \leq 0.03 \quad \text{formula (1)}$$

In the formula, T21 is a width of the tapered structure at a portion closest to the slit opening, T22 is a width of the tapered structure at a portion farthest from the slit opening, and T11 is a length of the tapered structure.

When the slit of the fixture of the present invention has the bent structure as the fixing structure, the slit is configured such that the slit linearly extends for a certain distance from the slit opening into which a cryopreservation jig is inserted toward the inside of the base, and then the slit bends in a direction different from the linearly extending direction. When the slit has such a bent structure, the cryopreservation jig inserted from the slit opening is further linearly inserted in a direction different from the direction which linearly extends from the opening, whereby the cryopreservation jig is fixed through the bent structure. Advantageously, this can prevent sudden slipping of the cryopreservation jig toward the opening. Preferably, the certain distance that the slit linearly extends toward the inside of the base is at least three times the external length of the cap member to be fixed (the external length of the cap member in the insertion direction).

Of these fixing structures (I) to (III) described above, preferred is the tapered structure (II), which can reliably fix a cryopreservation jig and provides excellent handleability when fixing. The bent structure (III) capable of preventing sudden slipping of a cryopreservation jig toward the opening is also preferred. Further, the fixture in which the slit has the bent structure (III) in which the slit bends in a direction different from its linearly extending direction and the tapered structure (II) located after the bend is particularly preferred because such a fixture can prevent sudden slipping of a cryopreservation jig toward the opening and can also provide both excellent fixing stability of the cryopreservation jig and excellent handleability when fixing.

The shape of the fixture of the present invention (the approximate shape of the fixture, assuming that no slit is provided in the base or no insertion portion for inserting a cryopreservation jig is provided on the upper portion of the base, and no liquid surface gauge portion (described later) is attached) may be a prism such as a triangular prism, a quadrangular prism, or a hexagonal prism. The shape may also be a partial pyramid such as a triangular pyramid with a top surface or a quadrangular pyramid with a top surface. A prism is preferred, and a quadrangular prism is more preferred. The shape can also be a cylinder or a partial cone, for example.

In the present invention, preferably, the base is formed from a material resistant to liquid nitrogen. Preferred examples of such a material include various metals such as aluminum, iron, copper, and stainless steel alloy, ABS resin, acrylic resin, polypropylene resin, polystyrene resin, polyethylene resin, fluororesin, various engineering plastics, glass, and rubber materials. Metals are particularly preferred because they have excellent durability and they can suitably fix a cryopreservation jig owing to their own heavy weight. Of these, aluminum is preferred in terms of processability. The fixture of the present invention may be made of one or more types of materials.

Preferably, the fixture of the present invention includes a liquid surface gauge portion on the upper surface or one of the side surfaces of the base, the liquid surface gauge portion extending above the upper surface of the base. The liquid surface gauge portion of the fixture of the present invention is a structure used for controlling the liquid surface of a coolant. the liquid surface gauge portion makes it possible to easily adjust the liquid surface of the coolant. In other words, when a cryopreservation jig holding a cell or tissue is fixed to the fixture of the present invention, the liquid surface gauge portion makes it possible to easily confirm that the cell or tissue deposited on the cryopreservation jig is in a cooled state.

Preferably, the liquid surface gauge portion, which is preferably included in the fixture of the present invention, has a chimney shape (a prism shape such as a quadrangular prism or a cylindrical shape) extending from the bottom of the container toward the liquid surface of the coolant. As is the case with the base described above, preferably, the liquid surface gauge portion is formed from a material resistant to liquid nitrogen.

The liquid surface gauge portion of the fixture of the present invention may have any shape as long as the liquid surface gauge portion extends above the upper surface of the base and provides a reference for controlling the liquid surface of the coolant. Yet, preferably, the liquid surface gauge is out of the liquid surface of the coolant or is slightly hidden below the liquid surface, so that an operator can easily visually check the level of the liquid surface.

When the liquid surface gauge portion of the fixture of the present invention has a height that protrudes from the liquid surface of the coolant, preferably, the liquid surface gauge portion has a marking. The marking may be in any form, but preferably, it is a line segment parallel to the liquid surface of the coolant. Since the marking provides a reference for height of the liquid surface of the coolant, the liquid surface gauge only needs to have one marking or may have two or more markings in the height direction.

In a series of processes from cell or tissue cryopreservation to thawing, the fixture of the present invention can be suitably used particularly in refrigeration of a frozen cell or tissue taken out from a tank containing a coolant (typically, liquid nitrogen), and in thawing of the cell or tissue. Generally, when a cell or tissue is preserved by the vitrification method, a cryopreservation jig including a strip is used for cryopreservation. Such cryopreservation jigs are disclosed in Patent Literatures 7 to 11 described above and in WO 2011/070973, for example.

In the series of processes from cryopreservation to thawing, first, freezing is performed in which an equilibrated cell or tissue is dropped with a preservation solution to the strip of the cryopreservation jig, and then a coolant (preferably, liquid nitrogen) is used to cool the cell or tissue. Second, the frozen cell or tissue is preserved inside a coolant tank for a long time while a cryogenic environment is maintained. Third, thawing is performed in which the frozen cell or tissue is transferred from the cryogenic environment to a solution called a thawing solution, and is thawed and defrosted in the thawing solution. The fixture of the present invention can be suitably used to fix and hold the cryopreservation jig in the refrigeration and thawing of the cell or tissue taken out from the coolant tank.

In thawing in the series of processes from cryopreservation to thawing using the fixture of the present invention, the cryopreservation jig maintained in the vitrified state in the coolant tank is taken out from the tank, and is refrigerated in a coolant using the fixture of the present invention prior to transfer of the cell or tissue to the thawing solution. Here, the cell or tissue can be rapidly transferred to the thawing solution when the cryopreservation jig is held by the fixture of the present invention such that the tip of the tubular storage member or the tip of the cap member of the cryopreservation jig (i.e., the frozen cell or tissue) is below a liquid surface of the coolant and a portion of the cryopreservation jig is above the liquid surface of the coolant. Thus, the present invention can prevent the slowdown of the thawing rate of the frozen cell or tissue.

In the series of processes from cell or tissue cryopreservation to thawing, the fixture of the present invention can be suitably used in what is called a "closed-type series of processes from cryopreservation to thawing" in which a cell or tissue does not contact a coolant.

In freezing in the closed-type series of processes from cryopreservation to thawing, after a cell or tissue is dropped with a preservation solution to the strip, the cryopreservation jig is covered by a tubular storage member whose ends are completely blocked and is thus shielded from the outside environment. Alternatively, after a cell or tissue is dropped with a preservation solution to the strip, the strip is covered by the cap member and is thus shielded from the outside environment. After the shielding, the cryopreservation jig is immersed in a coolant (preferably, liquid nitrogen), whereby the cell or tissue is cooled and frozen. In freezing, storing, and the like in the above-described closed-type series of processes, the air and the preservation solution surrounding the cell or tissue is cooled and the vitrification state is maintained via the tubular storage member or the cap member.

After the vitrification state is maintained, the lid fixed to the tubular storage member above the liquid surface of the liquid nitrogen is removed, whereby the cryopreservation jig holding the cell or tissue can be rapidly taken out without contact with the liquid nitrogen and immersed in the thawing solution. Alternatively, the cryopreservation jig including the strip holding the cell or tissue is pulled out from the cap member fixed to the fixture of the present invention, whereby the cell or tissue held on the strip can be rapidly taken out without contact with the liquid nitrogen and immersed in the thawing solution. The latter cryopreservation jig is preferred for a faster operation.

The cell or tissue to be immersed in the thawing solution by the above-described operation is immersed in the thawing solution preferably within five minutes, more preferably within one minute, after the lid is removed from the tubular storage member. When pulling out the cryopreservation jig including the strip from the cap member, the cryopreservation jig is immersed in the thawing solution preferably within five minutes, more preferably within one minute, after the fitting is loosened. When the sealed state is released for a long time (for example, when a long time has elapsed after the lid is removed from the tubular storage member or after the fitting with the cap member is loosened for pulling out the cryopreservation jig from the cap member), a gas such as nitrogen or oxygen in the air may enter the tubular storage member or the cap member, and then the gas may be cooled and liquefied. In some cases, the liquefied gas is attached to the deposition part and brought into the thawing solution, decreasing the temperature of the thawing solution. Disadvantageously, a decrease in the temperature of the thawing solution causes the slowdown of the thawing rate.

In the series of processes from cell or tissue cryopreservation to thawing, the fixture of the present invention can also be used in what is called an "open-type series of processes from cryopreservation to thawing" in which a cell or tissue contacts liquid nitrogen. Also in the open-type series of processes, when the cryopreservation jig is fixed and held using the fixture of the present invention prior to transfer of a frozen cell or tissue to the thawing solution, advantageously, the cell or tissue can be rapidly transferred from the liquid nitrogen to the thawing solution.

In the above-described method in which the cryopreservation jig is entirely housed in the tubular storage container, a cutting tool and/or an extraction tool may be required in the thawing step, or it may be complicated to take out the housed cryopreservation jig.

Because of the above reasons, a cryopreservation jig at least including a body including a deposition part and a cap member freely attachable to and detachable from the body is preferred; and a method of freezing and thawing a cell or tissue is preferred which at least includes the following steps: a sealing step of depositing a cell or tissue with a preservation solution on the deposition part of the cryopreservation jig, and sealing the deposition part with the cap member; a freezing step of cooling the sealed deposition part by a coolant to freeze the cell or tissue; a refrigerating step of maintaining the frozen state of the cell or tissue frozen in the freezing step; and a thawing step of thawing the cell or tissue maintained in the frozen state, wherein prior to the thawing step, the cell or tissue sealed with the cap member is fixed to the fixing structure of the cryopreservation jig fixture according to any one of (1) to (5) above, and the cryopreservation jig is temporarily held in a state where the cell or tissue is below a liquid surface of the coolant and a joint portion between the body and the cap member is above the liquid surface of the coolant, and in the thawing step, the body and the cap member are separated from each other, and the deposition part of the body is immersed in a thawing solution to thaw the cell or tissue.

When the deposition part includes a preservation solution absorber, advantageously, an excess preservation solution can be effectively removed, resulting in high handleability and less preservation solution surrounding the cell or tissue. This achieves a high freezing rate and a high thawing rate.

In the freezing and thawing method of the present invention, first, a cell or tissue is deposited with a preservation solution on the deposition part of the above-described cryopreservation jig. Preferably, the operation is performed under transmission microscope observation (hereinafter also simply described as "under microscope observation"). Here, when the deposition part of the cryopreservation jig includes a preservation solution absorber, advantageously, an excess preservation solution can be effectively removed, resulting in high handleability and less preservation solution surrounding the cell or tissue. This achieves a high freezing rate and a high thawing rate.

Second, the deposition part of the body is inserted into the cap member so that the cap member is fitted and fixed. The deposition part may be inserted into the cap member under microscope observation. The deposition part inserted into the cap member is completely covered by the cap member, and the cap member is fitted and fixed to a fitting structural member of the body to complete sealing.

Then, the cap member is immersed in a coolant such as liquid nitrogen, and the cell or tissue deposited on the deposition part is cooled and frozen. Here, the deposition part covered by the cap member and completely sealed, and the cell or tissue on the deposition part are cooled without contact with a coolant.

Preferably, it takes not more than one minute from when the cell or tissue is deposited with the preservation solution on the deposition part and the deposition part is sealed to when the body sealed with the cap member in the sealing step is immersed in the coolant. More preferably, it takes not more than 30 seconds.

The cooled cell or tissue is refrigerated in a cold storage container maintained at a cryogenic temperature by a coolant or the like, while the vitrification state of the cell or tissue is maintained.

In the freezing and thawing method of the present invention, prior to the thawing step, the cryopreservation jig is temporarily held in a state in which the cell or tissue sealed with the cap member is below a liquid surface of the coolant and a joint portion of the cap member of the cryopreservation jig is above the liquid surface of the coolant. Here, preferably, the cryopreservation jig is held using a fixture having a function to fix the cap member in order to improve handleability.

In the freezing and thawing method of the present invention, prior to the thawing step, the cryopreservation jig is temporarily held in a state in which the cell or tissue sealed with the cap member of the cryopreservation jig is inside the coolant while a joint portion between the body and the cap member is outside the coolant. Subsequently, the fitting and fixing between the cap member and the body is loosened, and the cap member is then separated from the body. Then, the deposition part is immersed in a thawing solution (thawing step). These operations prior to the thawing step allow the deposition part of the body to be rapidly immersed in the thawing solution without contact with a coolant such as liquid nitrogen. Preferably, it takes not more than five minutes from when the fitting and fixing between the body and the cap member is loosened to when the deposition part is immersed in the thawing solution. More preferably, it takes not more than one minute. When the fitting and fixing is loosened and the sealed state is released for a long time, a gas such as nitrogen or oxygen may enter the cap member, and then the gas may be cooled and liquefied. Disadvantageously, the liquefied gas which is attached to the deposition part and brought into the thawing solution may decrease the temperature of the thawing solution.

The freezing and thawing method of the present invention has been described so far. Next, the cryopreservation jig used in the present invention and the fixture of the present invention are described in further detail with reference to the drawings.

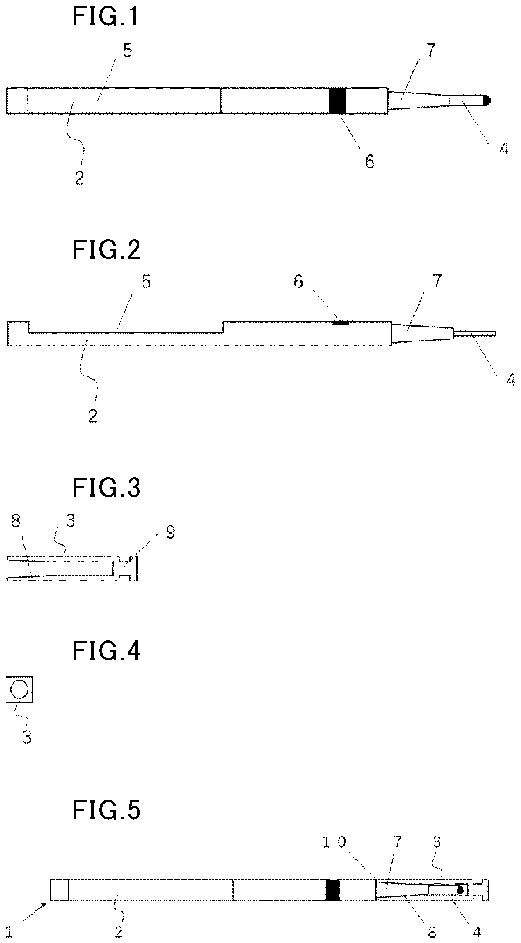

Fig. 1 is a schematic top view of an example of the cryopreservation jig to be fixed with the fixture of the present invention. A body 2 illustrated in Fig. 1 includes a deposition part 4, and the deposition part 4 is attached to a handle 5. The handle 5 has a marking 6 for distinguishing a side on which a cell or tissue is to be deposited. As illustrated in Fig. 1, the deposition part 4 may also include a marking. The body 2 includes a fitting structural member 7 for fixing the cap member to the body 2. In Fig. 1, the fitting structural member 7 has a tapered structure.

In the present invention, preferably, the handle has a prism shape in order to improve grippability and handleability. Preferably, the handle is a member formed from a material resistant to coolants such as liquid nitrogen. Preferred examples of such a material include various metals such as aluminum, iron, copper, and stainless steel, ABS resin, acrylic resin, polypropylene resin, polyethylene resin, fluororesin, various engineering plastics, and glass.

Fig. 2 is a schematic side view of an example of the cryopreservation jig to be fixed with the fixture of the present invention. The handle 5 of the body 2 illustrated in Fig. 2 includes a recess at a portion thereof, which facilitates distinguishing between the front and the back of the deposition part 4, i.e., a side on which a cell or tissue is to be deposited, when an operator grips the body 2.

Fig. 3 is a side cross-sectional view of an example of the cap member of the cryopreservation jig to be fixed with the fixture of the present invention. Specifically, it is an exemplary cap member that is suitable when the slit of the fixture of the present invention includes, as the fixing structure, (I) the irregular structure formed on at least one inner side surface of the slit. Fig. 3 also illustrates the internal structure of a cap member 3. The cap member 3 has a structure in which only one side is open. A fitting contact portion 8 for fixing the cap member 3 to the body is formed in the vicinity of the opening. The cap member 3 also includes a fixing portion 9 for fixing the cap member 3 to the fixture prior to the thawing step.

The cap member 3 may not necessarily include the fixing portion 9 when the fixture of the present invention has the following (II) or (III) as the fixing structure: (II) the tapered structure in which a distance between inner side surfaces of the slit gradually decreases toward a back of the slit; or (III) the bent structure in which the slit extends linearly from a slit opening toward an inside of the base and then bends in a direction different from the linearly extending direction.

Fig. 4 is a side view of an example of the cap member of the cryopreservation jig to be fixed with the fixture of the present invention, as viewed from an opening side. As illustrated in Fig. 4, the cap member 3 has an external shape in the form of a quadrangular prism, inside of which is a hollow having a circular truncated cone-shaped inner surface.

Fig. 5 is a schematic view of an example of the body and the cap member being fitted together and fixed. In Fig. 5 and some of the subsequent figures, the internal structure of the cap member 3 is illustrated to show the state of the deposition part 4 covered by the cap member 3. In a cryopreservation jig 1 illustrated in Fig. 5, the cap member 3 is completely fitted and fixed to the body 2 by the fitting contact portion 8 between the fitting structural member 7 of the body 2 and the cap member 3, and the deposition part 4 is covered and sealed with the cap member 3 and is thus protected. The boundary between the cap member 3 and the body 2 is a joint portion 10.

The tubular storage member and the cap member described above can be formed from materials, such as various resins and metals, which are resistant to coolants such as liquid nitrogen. The cap member may be made of one type or two or more types of materials. Particularly preferred is a resin because a tapered structure or a threaded structure can be easily formed by a process such as injection molding. Specific examples of the resin include polyester resins such as polyethylene terephthalate and polyethylene naphthalate, acrylic resin, epoxy resin, silicone resin, polycarbonate resin, diacetate resin, triacetate resin, polyacrylate resin, polyvinyl chloride resin, polysulfone resin, polyether sulfone resin, polyimide resin, polyamide resin, polyolefin resin, and cyclic polyolefin resin. When the cap member has a total light transmittance of 80% or more, advantageously, the state of the deposition part inside the cap member can be easily checked after the cap member is fitted.

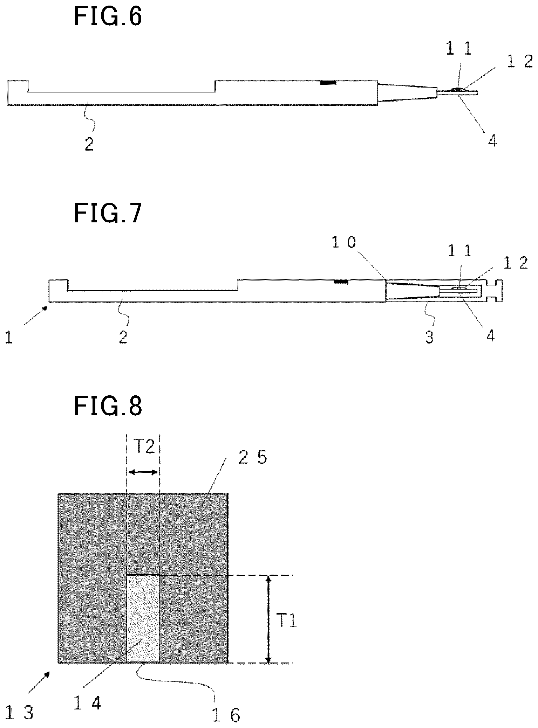

Fig. 6 is a schematic side view of a cell and a preservation solution being deposited on the cryopreservation jig. In the freezing and thawing method of the present invention, an operator drops a cell 11 and a preservation solution 12 to the deposition part 4 attached to the body 2 to deposit the cell 11 on the deposition part 4.

Fig. 7 is a schematic side view of the cap member being fitted and fixed after the cell and the preservation solution are deposited on the cryopreservation jig. The cell 11 is dropped with the preservation solution 12 to the deposition part 4 of the body 2 and is deposited on the deposition part 4. Then, the cap member 3 is fitted and fixed to the body 2. Thus, the cell 11 deposited on the deposition part 4 is completely covered and sealed with the cap member 3 and completely protected from the outside environment.

Preferably, the deposition part 4 of the body 2 described above has a strip shape. The deposition part having a strip shape can be easily covered and protected by the cap member. The shape of the deposition part 4 of the body 2 can be a flat planar sheet, a curved sheet, or a V-shaped sheet, for example. The flat planar sheet is preferred because it provides high working efficiency in depositing a cell or tissue on the deposition part.

In the present invention, examples of the deposition part of the cryopreservation jig include various resin films, metal plates, glass plates, and rubber plates. The deposition part may be made of one type or two or more types of materials. Particularly preferred is a resin film in terms of handling. Specific examples of the resin film include resin films made of polyester resins such as polyethylene terephthalate and polyethylene naphthalate, acrylic resin, epoxy resin, silicone resin, polycarbonate resin, diacetate resin, triacetate resin, polyacrylate resin, polyvinyl chloride resin, polysulfone resin, polyether sulfone resin, polyimide resin, polyamide resin, polyolefin resin, and cyclic polyolefin resin. When the deposition part has a total light transmittance of 80% or more, advantageously, the cell or tissue deposited on the deposition part can be easily checked under a transmission microscope.

In the present invention, the deposition part of the cryopreservation jig may preferably be a metal plate because it has excellent thermal conductivity and enables rapid freezing. Specific examples of the metal plate include copper, copper alloy, aluminum, aluminum alloy, gold, gold alloy, silver, silver alloy, iron, and stainless steel. Preferably, the various resin films, metal plates, glass plates, rubber plates, and the like described above each have a thickness of 10 $\mu$m to 10 mm. Depending on the purpose, it is possible to hydrophilize surfaces of these various resin films, metal plates, glass plates, rubber plates, and the like by an electrical method such as corona discharge treatment or a chemical method, and further to roughen these surfaces.

In the present invention, the deposition part of the cryopreservation jig can be a preservation solution absorber. When the deposition part is a preservation solution absorber, the preservation solution absorber can effectively remove an excess preservation solution, which improves handleability during deposition and freezing of a cell or tissue.

Examples of the preservation solution absorber described above include films made of wire mesh, paper, synthetic resin, or the like and having through holes. Another example of the preservation solution absorber may be a porous structure formed from a material having a refractive index of 1.45 or less. Owing to the porous structure, the preservation solution surrounding the cell or tissue can be removed. The porous structure also makes it possible to deposit and freeze a cell or tissue and thaw the cell or tissue after freezing with good visibility in an easy and reliable manner under transmission optical microscope observation.

The refractive index of the material of the porous structure described above can be measured using, for example, an Abbe's refractometer (Na light source; wavelength: 589 nm) in accordance with JIS K 0062:1992 and JIS K 7142:2014. Examples of the material having a refractive index of 1.45 or less to form the porous structure include plastic resin materials such as silicone resin and fluororesin (e.g., polytetrafluoroethylene resin, polyvinylidene difluoride resin, and polychlorotrifluoroethylene resin), metal oxide materials such as silicon dioxide, and inorganic materials such as sodium fluoride, magnesium fluoride, and calcium fluoride.

The pore size of the preservation solution absorber made of the porous structure is preferably 5.5 $\mu$m or less, more preferably 1.0 $\mu$m or less, still more preferably 0.75 $\mu$m or less. This can improve visibility of the cell or tissue under optical microscope observation. The thickness of the preservation solution absorber is preferably 10 to 500 $\mu$m, more preferably 25 to 150 $\mu$m. When the preservation solution absorber is a porous structure made of a plastic resin material, the pore size of the preservation solution absorber is the diameter of the largest pore measured by the bubble point test. When the preservation solution absorber is a porous structure made of a metal oxide or an inorganic material, the pore size is the average pore diameter determined by image observation of the surface and cross section of the porous structure.

The porosity of the preservation solution absorber is preferably 30% or more, more preferably 70% or more. The porosity is defined by a formula shown below. To determine the void volume V, a mercury porosimeter (name: Autopore II 9220, Micromeritics Instrument Corporation) is used to measure and process the cumulative pore volume (mL/g) of pores having a pore radius of 3 nm to 400 nm in the preservation solution absorber, and the cumulative pore volume (mL/g) is multiplied by the dry solids content (g/m$^2$) of the preservation solution absorber, whereby the void volume V can be determined as the value per unit area (m$^2$). The thickness T of the preservation solution absorber can be measured on a photograph of the cross section of the preservation solution absorber taken with an electron microscope.

$$P = (V/T) \times 100 \ (\%)$$

P: porosity (%)
V: void volume (ml/m$^2$)
T: thickness ($\mu$m)

Fig. 8 is a schematic top view of an example of the fixture of the present invention. In Fig. 8, a fixture 13 includes one slit 14 into which the cryopreservation jig is inserted on an upper surface of a base 25. The slit 14 includes a slit opening 16 at one end which opens at a lateral portion of the base 25. The other end of the slit 14 is blocked in the vicinity of a central portion of the upper surface of the base 25 in the figure. When inserting a cryopreservation jig (not illustrated) into the slit 14, the cryopreservation jig is inserted through the slit opening 16 and moved along the slit 14 toward the central portion of the upper surface of the base 25 in the figure.

T1 in Fig. 8 shows the length (in the horizontal direction) of the slit 14 of the fixture 13. T1 is not limited as long as it is long enough to allow a cryopreservation jig to be held after insertion. Yet, when freezing and thawing a cell or tissue by the Cryotop method described above, T1 is preferably 3 to 50 mm, more preferably 5 to 40 mm. Each dimension of the fixture 13 described in the following description is set with intention for freezing and thawing by the Cryotop method.

T2 in Fig. 8 shows the length of the slit 14 in the width direction. In the present invention, T2 is not limited as long as it is long enough to allow a cryopreservation jig to be inserted, fixed, and held. Yet, it is preferably 1 to 10 mm, more preferably 2 to 5 mm.

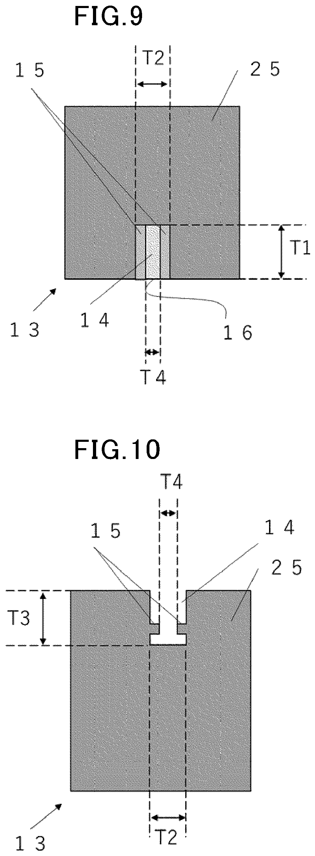

Fig. 9 is a schematic top view of another example of the fixture of the present invention. In Fig. 9, the fixture 13 includes one slit 14 with a groove structure into which a cryopreservation jig is inserted on the upper surface of the base 25. The slit 14 includes the slit opening 16 at one end which opens at a lateral portion of the base 25. The other end of the slit 14 is blocked in the vicinity of the central portion of the upper surface of the base 25 in the figure. The slit 14 includes an irregular structure 15 on each inner side surface thereof for fixing a cryopreservation jig. With such a structure, it is possible to insert the cap member 3 (not illustrated) through the slit opening 16, thrust the cap member 3 by sliding it toward the blocked portion, and reliably fix the cap member 3 at the blocked end. Fig. 9 illustrates a protruding irregular structure. When inserting a cryopreservation jig (not illustrated) into the slit 14, the cryopreservation jig is inserted through the slit opening 16 and moved along the slit 14 toward the central portion of the upper surface of the base 25.

T1 in Fig. 9 shows the length (in the horizontal direction) of the slit 14 of the fixture 13. T1 is not limited as long as it is long enough to allow a cryopreservation jig to be held after insertion. Yet, as described above, T1 is preferably 3 to 50 mm, more preferably 5 to 40 mm.

T2 in Fig. 9 shows the length of the slit 14 in the width direction. In the present invention, T2 is not limited as long as it is long enough to allow a cryopreservation jig to be inserted, fixed, and held. Yet, it is preferably 1 to 10 mm, more preferably 2 to 5 mm.

T4 in Fig. 9 shows the length of a portion narrowed by the irregular structures 15 of the slit 14 of the fixture 13 in the width direction. In the present invention, T4 is not limited as long as it is long enough to allow a cryopreservation jig (not illustrated) to be fixed and held. Yet, it is preferably 0.5 to 5 mm, more preferably 1.5 to 4 mm.

Fig. 10 is a schematic side view of an example of the irregular structure of the fixture (the fixture in Fig. 9) of the present invention. In Fig. 10, the fixture 13 includes the slit 14 into which a cryopreservation jig is inserted on the upper surface (top surface) of the base 25, and the slit 14 includes the irregular structure 15 on each inner side surface thereof for fixing the cryopreservation jig. In Fig. 10, each irregular structure 15 of the slit of the fixture is a protruding structure. As is the case with Fig. 9, T2 shows the length of the slit 14 in the width direction, and T4 shows the length of a portion narrowed by the protruding irregular structures 15 of the slit 14 in the width direction.

T3 in Fig. 10 shows the length of the slit 14 of the fixture 13 in the depth direction. In the present invention, T3 is not limited as long as it is long enough to allow a cryopreservation jig (not illustrated) to be fixed, and held. Yet, it is preferably 3 to 50 mm, more preferably 5 to 20 mm.

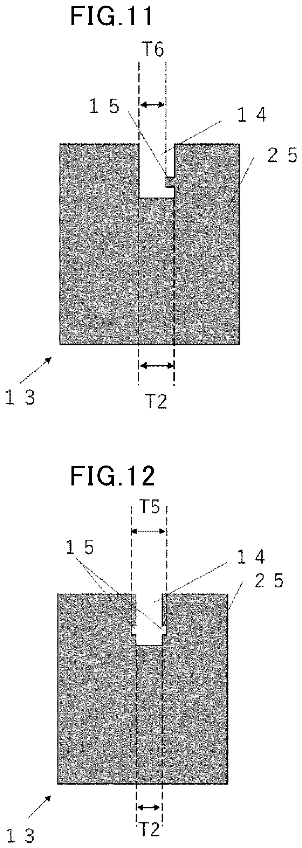

Fig. 11 is a schematic side view of another example of the irregular structure of the fixture of the present invention. In Fig. 11, the slit 14 of the fixture 13 includes one protruding irregular structure 15 for fixing a cryopreservation jig only on one inner side surface of the slit 14. T6 in Fig. 11 shows the length of a portion narrowed by one protruding irregular structure 15. With the use of the fixture having such a shape, a cryopreservation jig can be easily inserted and moved along the slit 14 of the fixture 13. Fig. 10 and Fig. 11 each only show the shape of the side surface of the base, and a blocked surface that would be visible inside the slit and the like are omitted.

Fig. 12 is a schematic side view of still another example of the irregular structure of the fixture of the present invention. In Fig. 12, the slit 14 of the fixture 13 includes the irregular structure 15 for fixing a cryopreservation jig on each inner side surface thereof. In Fig. 12, the irregular structures 15 of the fixture are recesses.

T5 in Fig. 12 shows the length of a portion of the slit expanded by the recessed irregular structures 15 of the slit 14 in the width direction. The length T5 is not limited as long as it is long enough to allow a cryopreservation jig (not illustrated) to be fixed and held. Yet, it is preferably 2 to 11 mm, more preferably 2.5 to 5.5 mm. As illustrated in Fig. 12, when the slit 14 includes the recessed irregular structures 15, the length T5 of the portion expanded by the recessed irregular structures 15 in the width direction is greater than the length T2 of the slit 14 in the width direction described above.

In the present invention, the slit 14 may include multiple irregular structures illustrated in Fig. 10 (the protruding irregular structures in which protrusions are provided on both inner surfaces of the slit 14) or multiple irregular structures illustrated in Fig. 12 (the recessed irregular structures in which recesses are provided on both inner surfaces of the slit 14), for example. Alternatively, the slit 14 may include a combination of the protruding irregular structure and the recessed irregular structure described above.

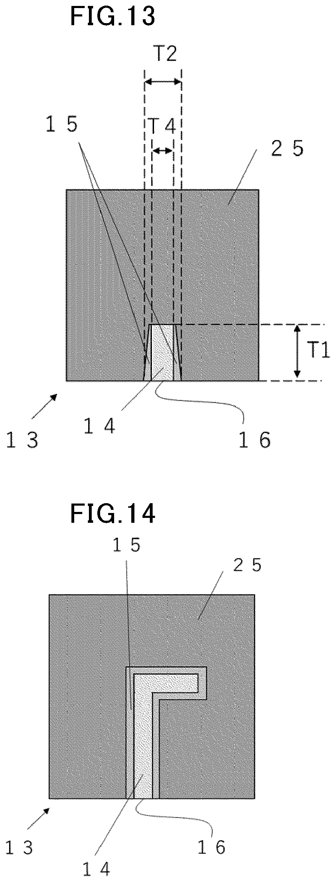

Fig. 13 is a schematic top view of another example of the fixture of the present invention. In Fig. 13, the slit 14 of the fixture 13 has the tapered structure and the irregular structure as the fixing structures for securely fixing a cryopreservation jig.

In Fig. 13, the length T2 of the slit 14 of the fixture 13 in the width direction gradually decreases in the insertion direction from the slit opening 16. Meanwhile, the width T4 narrowed by the protruding irregular structures 15 formed on both inner surfaces of the slit 14 is constant. With the use of the fixture 13 illustrated in Fig. 13, a cryopreservation jig inserted along the irregular structures 15 is fixed by the irregular structures 15 as well as by the walls of the slit 14 with their distance decreasing toward the back of the slit, so that the cryopreservation jig can be more securely fixed and held when inserted more deeply.

Fig. 14 is a schematic top view of still another example of the fixture of the present invention. In Fig. 14, the fixture 13 includes the slit 14 with the irregular structure 15, which bends at 90 degrees in the middle. With the use of the fixture having such a shape, when a cryopreservation jig is inserted into the slit 14 through the slit opening 16 and pushed all the way through the portion bent at 90 degrees, advantageously, the fixed and held cryopreservation jig can be prevented from suddenly slipping off from the slit opening 16.

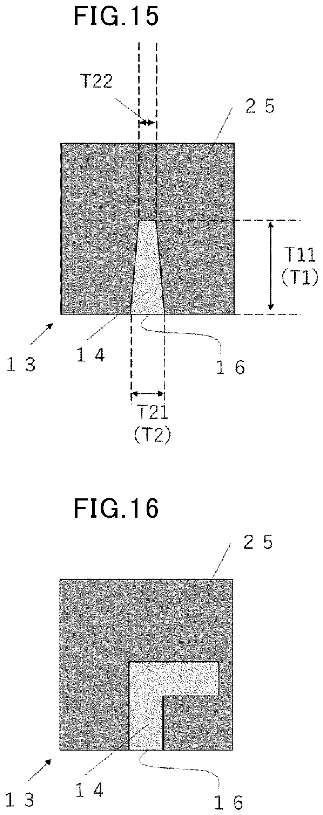

Fig. 15 is a schematic top view of still another example of the fixture of the present invention. In Fig. 15, the slit 14 of the fixture 13 has the tapered structure as the fixing structure for securely fixing a cryopreservation jig.

In Fig. 15, T21 shows the width of the tapered structure at a portion closest to the slit opening; T22 shows the width of the tapered structure at a portion furthest from the slit opening; and T11 shows the length of the tapered structure.

In Fig. 15, the tapered structure of the fixture 13 is a structure in which the distance between the side surfaces of the slit 14 gradually decreases from the slit opening 16 toward the central portion of the base 25, i.e., T21 > T22. As in the fixture illustrated in Fig. 15, when the slit 14 has the tapered structure, advantageously, the cryopreservation jig inserted through the slit opening 16 and moved along the slit 14 can be securely fixed. When the value of (T21 - T22)/T11 is 0.03 or lower, advantageously, the cryopreservation jig can be securely held, and the frozen cell or tissue can be rapidly transferred to a thawing solution kept warm. The value of (T21 - T22)/T11 described above is preferably 0.001 or higher in terms of fixing stability of the cryopreservation jig.

The fixture 13 in Fig. 15 is also an example in which the slit 14 only has the tapered structure. Thus, in Fig. 15, the width (T2) of the slit is the same as the width (T21) of the tapered structure at a portion closest to the slit opening, and length (T1) of the slit is the same as length (T11) of the tapered structure.

Fig. 16 is a schematic top view of still another example of the fixture of the present invention. In Fig. 16, the slit 14 of the fixture 13 has the bent structure as the fixing structure for securely fixing a cryopreservation jig. The slit 14 of the fixture in Fig. 16, which has the bent structure, has a shape in which the slit linearly extends for a certain distance from the slit opening 16 toward the central portion of the base 25 and then bends at 90 degrees. When the fixture including the bent slit is used, advantageously, the fixed and held cryopreservation jig can be prevented from suddenly slipping off from the slit opening 16.

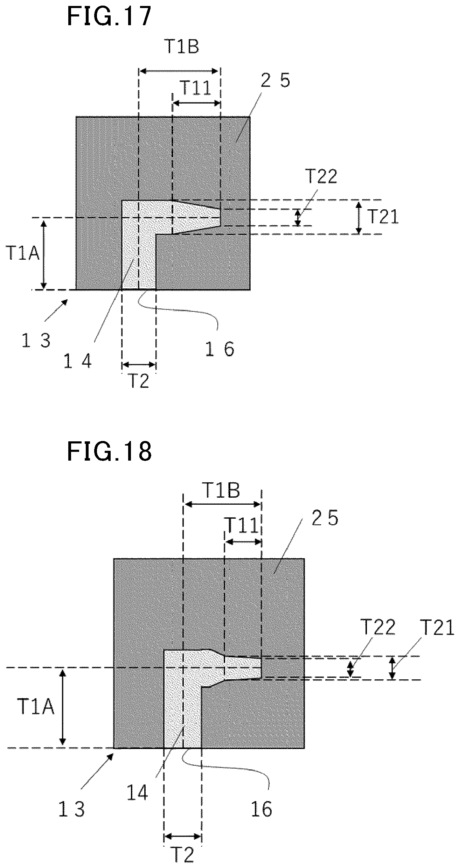

Fig. 17 is a schematic top view of still another example of the fixture of the present invention. In Fig. 17, the slit 14 of the fixture 13 has the bent structure and the tapered structure in the back of the slit, as the fixing structures for fixing a cryopreservation jig. In Fig. 17, the slit 14 of the fixture 13 has a shape in which the slit linearly extends for a certain distance (T1A) from the slit opening 16 toward the central portion of the base 25 and then bends at 90 degrees. The slit 14 also has the tapered structure after it bends to the right at 90 degrees. In Fig. 17, the width (T21) of the tapered structure at a portion closest to the slit opening and the width (T22) of the tapered structure at a portion furthest from the slit opening have a relationship of T21 > T22.

In Fig. 17, T1B is the distance from the point where the slit 14 bends at 90 degrees to the point furthest from the slit opening 16.

When the fixture having the shape illustrated in Fig. 17 is used, a cryopreservation jig is inserted into the slit 14 through the slit opening 16, passed through the portion bent at 90 degrees, and further inserted toward the blocked surface of the slit, whereby the cryopreservation jig can be fixed by the tapered structure. When the slit has both the bent structure and the tapered structure as the fixing structures, advantageously, even if the cryopreservation jig comes off from the tapered structure, the cryopreservation jig can still be held by the bent structure.

Fig. 18 is a schematic top view of still another example of the fixture of the present invention. In Fig. 18, the slit 14 of the fixture 13 has both the bent structure and the tapered structure as the fixing structures for fixing a cryopreservation jig. The fixture illustrated in Fig. 18 includes a tapered guide portion and the tapered structure for fixing a cryopreservation jig. In such a case, preferably, the tapered structure in which its width (T21) at a portion closest to the slit opening and its width (T22) at a portion furthest from the slit opening satisfy the relationship of T21 > T22 is located in the back of the slit. In this case, advantageously, the cryopreservation jig can be securely fixed and held, and it is possible to improve working efficiency in inserting the cryopreservation jig into the tapered structure where the cryopreservation jig is actually fixed.

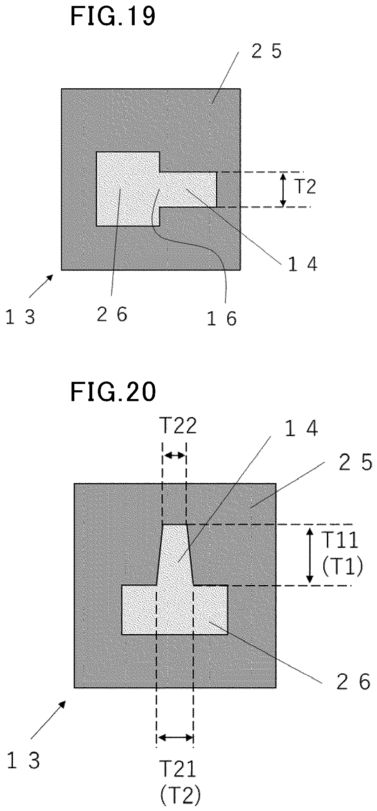

Fig. 19 is a schematic top view of still another example of the fixture of the present invention. In Fig. 19, the fixture 13 includes an insertion portion 26 into which a cryopreservation jig is inserted on the upper surface (top surface) of the base 25, and includes the slit opening 16 at a lateral portion of the insertion portion 26. The fixture as illustrated in Fig. 19 does not include an opening at a lateral portion of the base. Thus, a cryopreservation jig fixed and held by the fixture is less likely to suddenly slip off.

Fig. 20 is a schematic top view of still another example of the fixture of the present invention. In Fig. 20, the fixture 13 includes, on the upper surface of the base 25, the insertion portion 26 into which a cryopreservation jig is inserted, and the slit 14 whose one end is opened at a side surface of the insertion portion 26, wherein the slit 14 has the tapered structure for fixing a cryopreservation jig. When the fixture having such a shape is used, advantageously, the cryopreservation jig can be securely fixed by the tapered structure, and even when the fixed and held cryopreservation jig slips off from the slit opening, the cryopreservation jig can stay in the insertion portion 26.

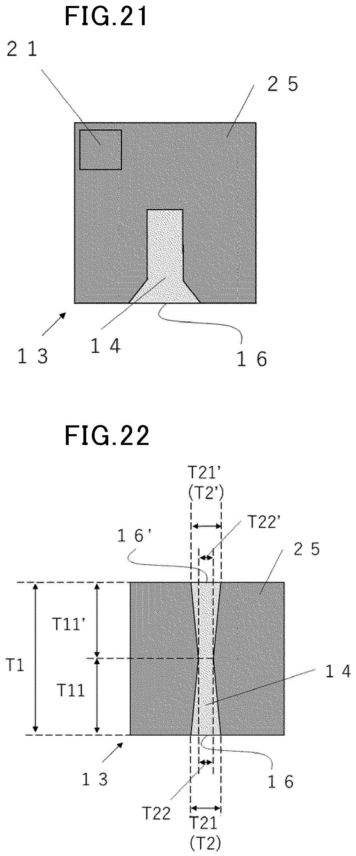

Fig. 21 is a schematic top view of still another example of the fixture of the present invention. In Fig. 21, the slit opening 16 of the fixture 13 is opened wider than the width of the slit 14, thus providing excellent working efficiency in inserting a cryopreservation jig. Fig. 21 illustrates a liquid surface gauge portion 21 having a quadrangular prism shape attached to the upper surface (top surface) of the base 25.

Fig. 22 is a schematic top view of still another example of the fixture of the present invention. In Fig. 22, the fixture 13 includes two slit openings (the slit opening 16 and a slit opening 16') in one slit 14. The slit 14 has tapered structures in each of which the width of a groove of the slit 14 decreases from the slit opening toward the central portion of the base 25. With the shape as illustrated in Fig. 22, a cryopreservation jig (not illustrated) can be inserted from either end of the slit 14, thus providing a fixture in which multiple cryopreservation jigs can be held simultaneously. In addition, in this case, a larger area of the fixed cryopreservation jig will contact a coolant, as compared to the case where the slit 14 is blocked. Thus, more efficient cooling can be expected.

The length T1 of the slit 14 is the total of the length T11 of the tapered structure including the slit opening 16 and the length T11' of the tapered structure including the slit opening 16'.

In the fixture illustrated in Fig. 22, the shapes of the two slit openings (i.e., the value of (T21 - T22)/T11 at the slit opening 16 and the value of (T21' - T22')/T11' at the slit opening 16') may be the same as or different from each other, but preferably, the same.

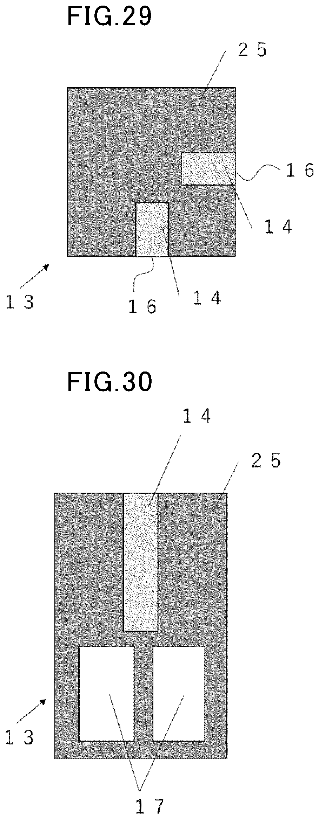

Fig. 22 described above illustrates one example in which two slit openings are provided in one slit. In other examples, it is also possible to provide slits (independent slits) on the upper surface of the base, each having a slit opening at a different lateral portion of the base 25 (Fig. 29 described later), and it is also possible to provide slits (independent slits) on the upper surface of the base, each having a slit opening at the same lateral portion of the base 25.

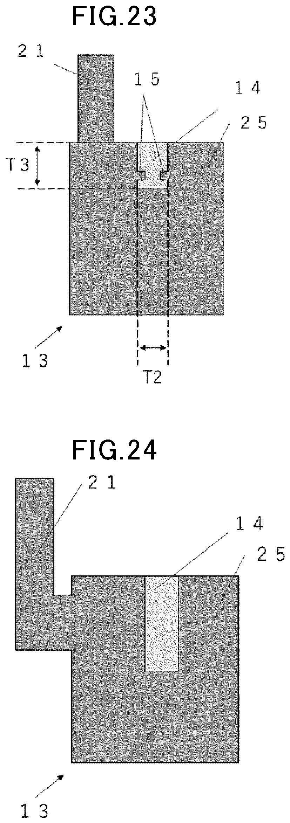

Fig. 23 is a schematic side view of still another example of the slit of the fixture of the present invention. In Fig. 23, the slit 14 of the fixture 13 includes the irregular structure 15 on each inner side surface thereof as the fixing structure for securely fixing a cryopreservation jig. In the fixture in Fig. 23, each irregular structure 15 is a protrusion (alternatively, each irregular structure may be a recess). The cryopreservation jig includes the liquid surface gauge portion 21 on the upper surface (top surface) of the base 25.

Fig. 24 is a schematic side view of still another example of the fixture of the present invention. In Fig. 24, the fixture 13 includes the slit 14 and the liquid surface gauge portion 21 attached to one of the side surfaces of the base 25 and having a structure that extends above the upper surface (top surface) of the base 25.

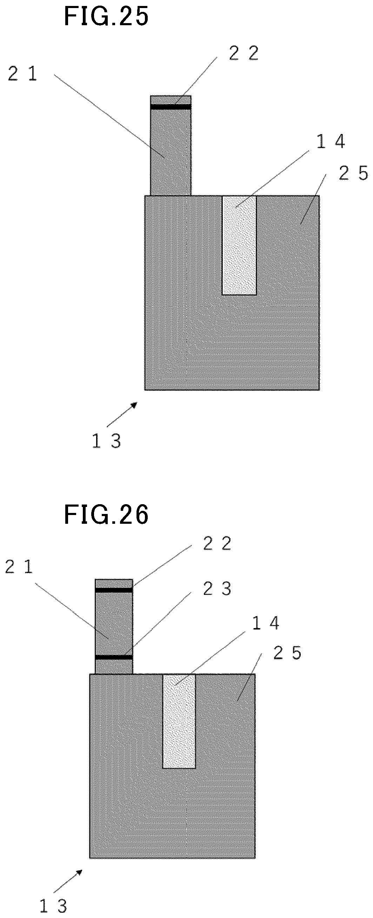

Fig. 25 is a schematic side view of still another example of the fixture of the present invention. In Fig. 25, the fixture 13 includes the slit 14 and the liquid surface gauge portion 21 having a quadrangular prism shape attached to the upper surface (top surface) of the base 25. In Fig. 25, the liquid surface gauge portion 21 of the fixture 13 includes one marking, and the marking illustrated in Fig. 25 is an upper limit marking 22. The upper limit marking 22 is a line segment parallel to a liquid surface of liquid nitrogen (not illustrated). An operator can easily check whether the frozen cell or tissue is reliably cooled by controlling the amount of liquid nitrogen using the position of the marking as a guide.

Fig. 26 is a schematic side view of still another example of the fixture of the present invention. In Fig. 26, the fixture 13 includes the slit 14 and the liquid surface gauge portion 21 having a quadrangular prism shape attached to the upper surface (top surface) of the base 25. In Fig. 26, the liquid surface gauge portion 21 of the fixture 13 includes a total of two markings including the upper limit marking 22 and a lower limit marking 23. When the fixture 13 includes the upper limit marking 22 and the lower limit marking 23, for example, an operator only needs to set and provide a liquid surface of liquid nitrogen between the upper limit marking 22 and the lower limit marking 23. This further facilitates control of the amount of liquid nitrogen.

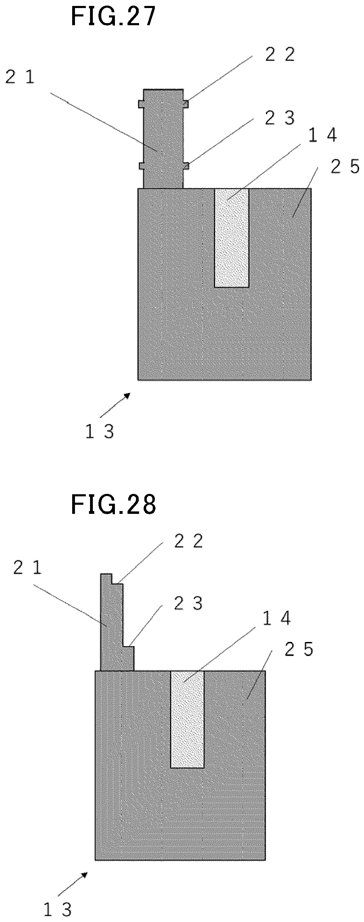

Fig. 27 is a schematic side view of still another example of the fixture of the present invention. In Fig. 27, the fixture 13 includes the slit 14 and the liquid surface gauge portion 21 having a quadrangular prism shape attached to the upper surface (top surface) of the base 25. In Fig. 27, the liquid surface gauge portion 21 of the fixture 13 includes the upper limit marking 22 having a protruding shape and the lower limit marking 23 having a protruding shape, and an operator can set and provide a liquid surface of liquid nitrogen between the upper limit marking 22 having a protruding shape and the lower limit marking 23 having a protruding shape.

Fig. 28 is a schematic side view of still another example of the fixture of the present invention. In Fig. 28, the fixture 13 includes the slit 14 and the liquid surface gauge portion 21 having a three-dimensional stepped shape attached to the upper surface (top surface) of the base 25. As is the case with the marking, an operator can set and provide a liquid surface of liquid nitrogen between the stepped upper limit marking 22 and the stepped lower limit marking 23.

Fig. 29 is a schematic top view of still another example of the fixture of the present invention. In Fig. 29, the fixture 13 includes multiple independent slits 14 on the upper surface of the base 25. The slits 14 each can fix a cryopreservation jig. According to the present embodiment, since the base 25 includes more slits 14 on its upper surface, many cryopreservation jigs can be fixed and held simultaneously.

Fig. 30 is a schematic side view of still another example of the fixture of the present invention. In Fig. 30, the fixture 13 includes hollow portions 17. When the fixture 13 includes the hollow portions 17, advantageously, the thermal capacity of the fixture 13 is reduced, and thus the amount of liquid nitrogen to be volatilized is reduced when the fixture 13 is immersed in liquid nitrogen.

The following describes in detail a method of freezing and thawing a cell or tissue using the fixture of the present invention.

In the present invention, a cell or tissue is deposited with a preservation solution on a deposition part of a cryopreservation jig at least including a body including the deposition part and a cap member freely attachable to and detachable from the body. The deposition part is sealed with the cap member. The sealed deposition part is then cooled by a coolant, and the frozen state of the frozen cell or tissue is maintained. Subsequently, the cell or tissue is thawed. For thawing of the cell or tissue, when the cryopreservation jig is temporarily held in a state in which the cell or tissue sealed with the cap member is below a liquid surface of the coolant and a joint portion between the body and the cap member is above the liquid surface of the coolant, advantageously, the body and the cap member can be quickly separated from each other, and the deposition part thus can be rapidly immersed in a thawing solution.

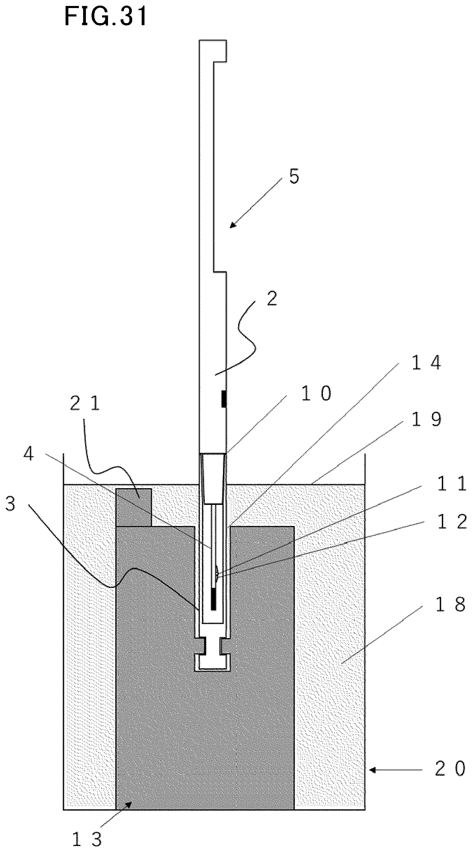

Fig. 31 is a schematic view of the cell or tissue sealed with the cap member being below a liquid surface of a coolant and a joint portion of the cap member being above the liquid surface of the coolant, prior to the thawing step. In Fig. 31, the cryopreservation jig at least includes the body 2 and the cap member 3 freely attachable to and detachable from the body 2, and the cell 11 is deposited with a very small amount of the preservation solution 12 on the deposition part 4 of the body 2.

The deposition part 4 holding the cell 11 with a very small amount of the preservation solution 12 is sealed with the cap member 3. The cell 11 deposited on the deposition part 4 is cooled because it is below a liquid surface 19 of liquid nitrogen 18, and the frozen cell 11 is maintained in a frozen state. When thawing the frozen cell 11, the joint portion 10 between the cap member 3 and the body 2 is above the liquid surface 19 of the liquid nitrogen 18. Thus, even when the fitting and fixing between the body 2 and the cap member 3 is loosened and the fitting is released, advantageously, the body 2 can be quickly pulled out and separated from the cap member 3 while the surrounding coolant (liquid nitrogen) is prevented from directly entering the cap member 3, and the deposition part can be rapidly immersed in a thawing solution. When the fixture 13 includes the liquid surface gauge portion 21, advantageously, an operator can easily know the position of the liquid surface 19, which can improve working efficiency. Fig. 31 illustrates an example of a case where the upper surface of the liquid surface gauge portion 21 attached to the upper surface (top surface) of the fixture 13 is used as a reference of the position of the liquid surface 19. The use of the liquid surface gauge portion 21 makes it easy to position the liquid surface 19 above the cell 11 and below the joint portion 10.

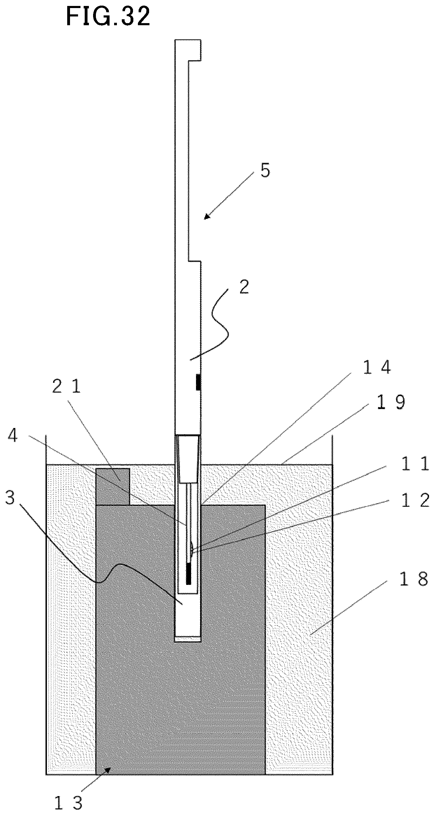

Fig. 32 is another schematic view of the cell or tissue sealed with the cap member being below the liquid surface of the coolant and the joint portion of the cap member being above the liquid surface of the coolant, prior to the thawing step. In Fig. 31 above, the cryopreservation jig is fixed by the fixture 13 including the slit with the irregular structure. Fig. 32 illustrates the cryopreservation jig being fixed by the fixture 13 including the slip with the tapered structure (not illustrated).

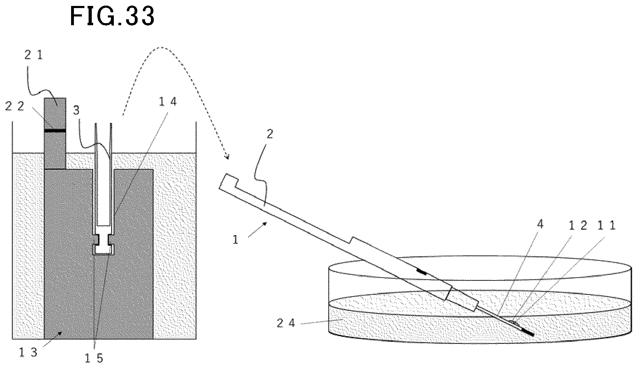

Fig. 33 is a schematic view of the body and the cap member being separated from each other, and the deposition part being immersed in a thawing solution. In Fig. 33, the cap member 3 is fixed to the fixture 13, so that the body 2 including the deposition part 4 can be rapidly separated from the cap member 3 by simply pulling out the body 2. After separation, the deposition part 4 is immersed in a thawing solution 24, and the cell 11 is thawed and recovered in the thawing solution 24.

When thawing the cell or tissue using the fixture of the present invention, the preservation solution may be one commonly used for freezing cells, such as eggs and embryos. For example, the preservation solution may be the above-described preservation solution prepared by adding a cryoprotectant (e.g., glycerol or ethylene glycol) to a physiological solution such as a phosphate buffered saline, or a preservation solution containing a large amount (at least 10 mass% or more, more preferably 20 mass% or more relative to the total mass of the preservation solution) of a cryoprotectant such as glycerol, ethylene glycol, or dimethyl sulfoxide (DMSO). The thawing solution may be one that is commonly used to thaw cells such as eggs and embryos. For example, a thawing solution prepared by adding 1 M sucrose for osmoregulation to the above-described physiological solution such as phosphate buffered saline may be used.

Examples of the cell that can be cryopreserved and thawed in the present invention include reproductive cells such as eggs, embryos, and sperms from mammals (for example, human, bovine, swine, equine, leporine, rat, and mouse); and pluripotent stem cells such as induced pluripotent stem cells (iPS cells) and embryonic stem cells (ES cells). Examples also include culture cells such as primary culture cells, subculture cells, and cell lines. In one or more embodiments, examples of the cell include adhesive cells such as fibroblasts, cancer-derived cells (e.g., pancreatic cancer cells and hepatoma cells), epithelial cells, vascular endothelial cells, lymphatic endothelial cells, neuronal cells, chondrocytes, tissue stem cells, and immune cells. Examples of the tissue that can be cryopreserved and thawed include tissues formed of homologous cells and tissues formed of heterologous cells, such as tissues of ovary, skin, corneal epithelium, periodontal ligament, and myocardium.

EXAMPLES

The present invention is specifically described in more detail below with reference to examples, but the present invention is not limited to the examples below.

(Example 1)

In the following, deposition of a cell or tissue, sealing, freezing, refrigerating, and thawing steps were performed by the following procedure, using a cryopreservation jig.

<Production of cryopreservation jig>

The body 2 and the cap member 3 in the forms illustrated in Fig. 1 to Fig. 4 were produced. The deposition part 4 of the body 2 was a polyethylene terephthalate resin film having a strip shape (width; 1.5 mm; length: 20 mm; thickness: 250 μm), and was attached to the handle 5 made of ABS resin. The cap member 3 was made of ABS resin and had the shape illustrated in Fig. 3 and Fig. 4. The fitting contact portion 8 constituting a portion of the internal structure of the cap member 3 had a tapered structure. The opening of the cap member 3 had a circular shape having a diameter of 2 mm. The external shape of the cap member 3 was a quadrangular prism with a base side of 3.1 mm. The cap member 3 included recesses each having a depth of 0.3 mm in the vicinity of a tip thereof.

<Deposition of cell or tissue, sealing, freezing, and refrigerating steps>

An equilibrated mouse 8-cell stage embryo was dropped with a preservation solution to the deposition part 4 of the cryopreservation jig using a pipette. After an excess preservation solution was removed, the deposition part 4 was inserted into the cap member 3 under transmission microscope observation, whereby the cap member 3 was fitted and fixed (sealing step: the state illustrated in Fig. 7). Subsequently, the cap member-side of the cryopreservation jig 1 was immersed in liquid nitrogen (freezing step). The preservation solution was prepared by using commercially available Medium 199 (Life Technologies) containing L-glutamine, phenol red, and 25 mM HEPES (4-(2-hydroxyethyl)-1-piperazineethanesulfonic acid) as a base solution and adding ethylene glycol (15 vol%), dimethyl sulfoxide (DMSO) (15 vol%), sucrose (0.5 M), and gentamicin (50 mg/L) to the base solution. The time from when the mouse 8-cell stage embryo was transferred from an equilibration solution to the preservation solution to when the mouse 8-cell stage embryo was immersed in the liquid nitrogen was one minute. The cryopreservation jig used in the freezing step was stored in the liquid nitrogen until the thawing step (refrigerating step).

<Thawing step>

The cryopreservation jig after the above steps was taken out, fixed in the slit of the fixture 13 made of aluminum in the form illustrated in Fig. 31, and temporarily held in a state where the joint portion 10 of the cap member 3 of the cryopreservation jig was above the liquid surface 19 of the liquid nitrogen 18 serving as a coolant. Then, fitting between the cap member 3 and the handle 5 was loosened, the body 2 was separated from the cap member 3, and the deposition part 4 of the body 2 was immersed in a thawing solution kept warm at 37°C. Subsequently, the embryo was recovered from the deposition part 4. It was possible to separate the body 2 from the cap member 3 within 10 seconds after the fitting of the cap member 3 was loosened, and it was possible to immerse the deposition part 4 in the thawing solution within one second after the body 2 was separated from the cap member 3. The thawing solution was prepared by using Medium 199 described above as a base solution and adding 1 M sucrose thereto.

(Example 2)

The series of steps of the freezing and thawing method was performed on a frozen mouse 8-cell stage embryo as in Example 1, except for applying a polyurethane adhesive to both ends (2 mm, each) of a polyethylene terephthalate resin film (width: 1.5 mm; length 20: mm; thickness: 180 $\mu$m) in the length direction and then bonding a porous resin sheet (pore size: 0.2 $\mu$m; porosity: 71%; thickness: 30 $\mu$m) made of hydrophilized polytetrafluoroethylene resin to the polyethylene terephthalate resin film to obtain the deposition part 4 of the cryopreservation jig. In the freezing and thawing method of Example 2, an excess preservation solution that was present when the embryo was dropped was automatically removed during deposition of the cell or tissue. Thus, there was no need to remove the excess preservation solution using a pipette.

(Comparative Example 1)

A freezing and thawing method of Comparative Example 1 was performed as in Example 1, except for directly immersing the deposition part 4 in the liquid nitrogen without the cap member 3 thereon after the mouse 8-cell stage embryo was dropped to the deposition part 4 and then covering and protecting the deposition part 4 by the cap member 3 in the liquid nitrogen for freezing.

(Comparative Example 2)

A freezing and thawing method of Comparative Example 2 was performed as in Example 2, except for directly immersing the deposition part 4 in the liquid nitrogen without the cap member 3 thereon after the mouse 8-cell stage embryo was dropped to the deposition part 4 and then covering and protecting the deposition part 4 by the cap member 3 in the liquid nitrogen for freezing.

(Comparative Example 3)

] In the thawing step of Example 1, while the joint portion 10 of the cap member 3 of the cryopreservation jig was in the liquid nitrogen (the state in which the joint portion 10 was below the liquid surface of the liquid nitrogen), the fitting between the cap member 3 and the handle 5 was loosened, and the body 2 was separated from the cap member 3. Then, the deposition part 4 of the body 2 was immersed in the thawing solution kept warm at 37°C.

<Evaluation of air bubble attachment in thawing step>

Each of the cryopreservation jigs of Examples 1 and 2 and Comparative Examples 1 to 3 was subjected to evaluation of the state of air bubble attachment to the deposition part at five seconds after the deposition part was immersed in the thawing solution in the thawing step. The evaluation was performed by counting the number of air bubbles (diameter: 30 $\mu$m or more) attached to the surface of the deposition part 4 in the region from 2 mm to 7 mm from the tip (area: width of 1.5 mm $\times$ length of 5 mm). In each freezing and thawing method, the evaluation was repeated three times. Table 1 shows the results.

[Table 1]

| | Number of air bubbles attached to deposition part (pieces) | | | |
|---|---|---|---|---|
| | 1st time | 2nd time | 3rd time | Average |
| Example 1 | 0 | 0 | 0 | 0 |
| Example 2 | 0 | 0 | 0 | 0 |
| Comparative Example 1 | 30 | 75 | 12 | 39 |
| Comparative Example 2 | 25 | 47 | 14 | 28.7 |
| Comparative Example 3 | 20 | 40 | 10 | 23.3 |

The above results show that the use of the freezing and thawing method of the present invention can reduce or prevent air bubble attachment to the deposition part and the cell or tissue during thawing, thus facilitating recovery of the cell or tissue. In addition, the cell or tissue can be thawed in a very short time, which is expected to lead to high viability.

In the following, each fixture in the thawing step was examined in detail.

(Example 3)

Aluminum was subjected to metal cutting to produce a fixture of Example 3 in the form including one slit 14 with the protruding irregular structures 15 (illustrated in Fig. 9 and Fig. 10). The base 25 had a size of 50 mm × 50 mm × 70 mm (length × width × height). The slit 14 had a width T2 of 3.2 mm, a length T1 of 10 mm from a lateral portion of the base 25, and a depth T3 of 10 mm. The irregular structures were protruding structures of 0.25 mm from the respective inner side surfaces of the slit. The portion narrowed by the protruding irregular structures 15 had a length T4 of 2.7 mm in the width direction.

(Example 4)

Aluminum was subjected to metal cutting to produce a fixture of Example 4 in the form including one slit 14 with the protruding irregular structures 15 in which the width T2 of the slit 14 gradually decreased in the insertion direction from the slit opening (illustrated in Fig. 13). The base 25 had a size of 50 mm × 50 mm × 70 mm (length × width × height). The slit 14 had a length T1 of 10 mm from a lateral portion of the base 25, and a depth T3 of 10 mm. The slit 14 had a width T2 of 4 mm at the slit opening 16. The width T2 decreased at a constant rate in the insertion direction from the slit opening 16, and was 3 mm at a blocked portion of the slit 14 in the vicinity of a central portion of the upper surface of the base. The portion narrowed by the irregular structures 15 had a constant length T4 of 2.7 mm in the width direction.

(Example 5)

Aluminum was subjected to metal cutting to produce a fixture of Example 5 in the form including the protruding irregular structure 15 only on one inner side surface of the slit (illustrated in Fig. 11). The base 25 had a size of 50 mm × 50 mm × 70 mm (length × width × height). The slit had a width T2 of 3.2 mm, a length T1 of 10 mm from a lateral portion of the base 25, and a depth T3 of 10 mm. The irregular structure 15 was a protruding structure of 0.25 mm from the inner side surface of the slit. The portion narrowed by one protruding irregular structure 15 had a length T6 of 2.95 mm.

(Example 6)

A fixture of Example 6 was produced as in Example 3, except for processing ABS resin by a sheet lamination 3D printer.

(Example 7)

A sheet lamination 3D printer was used with ABS resin to produce a fixture of Example 7 in the form including the bent structure and the protruding irregular structure 15 extending on the inner side surfaces of the slit (illustrated in Fig. 14). The base 25 had a size of 50 mm × 50 mm × 70 mm (length × width × height). The slit 14 had a width T2 of 3.2 mm and a depth T3 of 10 mm. The slit 14 was formed to extend linearly for a length of 20 mm in the insertion direction, turn to the right by 90 degrees, and extend linearly for a length of 10 mm. The irregular structure 15 was a protruding structure of 0.25 mm from each inner side surface of the slit 14. The portion narrowed by the protruding irregular structure 15 had a length T4 of 2.7 mm in the width direction.

(Example 8)

Aluminum was subjected to metal cutting to produce a fixture of Example 8 in the form including one slit 14 and the liquid surface gauge portion 21 having a quadrangular prism shape attached to the upper surface of the base 25 (illustrated in Fig. 23). The base 25 had a size of 50 mm × 50 mm × 70 mm (length × width × height). The slit 14 had a width T2 of 3.2 mm, a length T1 of 30 mm from a lateral portion of the base 25, and a depth T3 of 30 mm. The liquid surface gauge portion 21 was attached to the upper surface (top surface) of the base 25. The liquid surface gauge portion 21 had a size of 15 mm × 15 mm × 15 mm. The slit 14 included the irregular structures 15. The irregular structures 15 were protruding structures of 0.25 mm from the respective inner side surfaces of the slit 14. The portion narrowed by the protruding irregular structures 15 had a length T4 of 2.7 mm in the width direction.

(Example 9)

Aluminum was subjected to metal cutting to produce a fixture of Example 9 in the form including the slit 14 with the tapered structure (illustrated in Fig. 15) and the liquid surface gauge portion 21 having a quadrangular prism shape attached to the upper surface of the base as in Example 8 above. The size of the base was as described in Example 8 above. The slit 14 had a width T21 of 4 mm at the slit opening 16, a width T22 of 3 mm at the blocked side, and a length T1 (the length T11 of the tapered structure) of 30 mm from a lateral portion of the base 25. The slope (T21 - T22)/T11 of the tapered structure was 0.033.

(Example 10)

A fixture of Example 10 was produced as in Example 9, except for processing polyamide resin by a 3D printer.

(Example 11)

A polyamide resin was processed by a 3D printer to produce a fixture of Example 11 including the insertion portion 26 for inserting a cryopreservation jig on the upper surface (top surface) of the base 25 and the slit opening 16 at a lateral portion of the insertion portion 26 in the form illustrated in the schematic top view of Fig. 19, and also including a liquid surface gauge portion (not illustrated). The base 25 had a size of 50 mm × 50 mm × 70 mm (length × width × height). The insertion portion 26 had a size of 20 mm × 20 mm × 30 mm (length × width × depth). The slit 14 had a width T2 of 3.2 mm, a length of 20 mm from the slit opening 16, and a depth T3 of 30 mm. The slit 14 included the protruding structures each extending over a length of 20 mm on both side surfaces as the irregular structures. In Example 11, the irregular structures of the slit of the fixture were protruding structures of 0.25 mm from the respective inner side surfaces of the slit 14. The portion narrowed by the protruding irregular structures had a length T4 of 2.7 mm in the width direction. In Example 11, the fixture included a liquid surface gauge portion (not illustrated) attached to the upper surface (top surface) of the base 25, and the liquid surface gauge portion had a size of 15 mm × 15 mm × 15 mm.

<Evaluation of fixing condition of cryopreservation jig>

For each of the fixtures of Examples 3 to 11, a cryopreservation jig as illustrated in Fig. 31 was produced to evaluate the fixing condition of the cryopreservation jig. For the cryopreservation jig, a polyethylene terephthalate resin film having a strip shape (width: 1.5 mm; length: 20 mm; thickness: 190 $\mu$m) was used as the deposition part 4, and the film was bonded to the handle 5 made of ABS resin to produce the body 2. The cap member 3 was made of ABS resin. The external shape of the cap member 3 was a quadrangular prism with a base side of 3.1 mm. The cap member 3 included recesses each having a depth of 0.3 mm in the vicinity of a tip thereof. An equilibrated mouse 8-cell stage embryo was dropped with a preservation solution (0.1 $\mu$L) to the above-described cryopreservation jig under a transmission microscope. The preservation solution had a composition containing DMSO (15 vol%), ethylene glycol (15 vol%), and sucrose (17 mass%) in Medium 199 available from Sigma-Aldrich. Then, the strip including the deposition part was inserted into the cap member 3 under a transmission microscope, and was subsequently immersed in liquid nitrogen for freezing. Subsequently, the cap member-side of the cryopreservation jig was fixed to the slit 14 in the liquid nitrogen. In each of Examples 1 to 5, the liquid nitrogen 18 was introduced into a freezing container 20 using the liquid surface gauge portion 21 of the fixture as a reference such that the liquid surface gauge portion 21 was slightly below the liquid surface 19 of the liquid nitrogen 18 in the form illustrated in Fig. 31 for preparation. In each of Examples 3 to 11, the cap member-side of the cryopreservation jig was inserted into the slit 14 of the fixture 13, and the fixing and holding state in the form illustrated in Fig. 31 was evaluated based on the following criteria. Table 2 shows the results in "Evaluation of fixing condition during thawing".
AA: The fixing was particularly securely completed, and was particularly highly evaluated.
A: The fixing was securely completed with stability.
B: The fixing was stably completed.
C: The fixing was completed but was slightly unstable.

[Table 2]

|  | Evaluation of fixing condition during thawing |
|---|---|
| Example 3 | B |

(continued)

|  | Evaluation of fixing condition during thawing |
|---|---|
| Example 4 | A |
| Example 5 | C |
| Example 6 | B |
| Example 7 | A |
| Example 8 | B |
| Example 9 | B |
| Example 10 | B |
| Example 11 | B |

(Example 12)

Aluminum was subjected to metal cutting to produce a fixture of Example 12 in which the slit 14 had the tapered structure illustrated in Fig. 15. The base 25 had a size of 50 mm × 50 mm × 70 mm (length × width × height). The slit 14 had a width T2 of 4 mm at the slit opening 16, which was the same as the width T21 of the tapered structure of the slit 14 at a position closest to the surface of the base, and the slit 14 had a width T22 of 3 mm at the blocked side. The slit 14 had a length T1 of 30 mm from a lateral portion of the base 25, which was the same as the length T11 of the tapered structure, and the slit 14 had a depth of 30 mm. The value of (T21 - T22)/T11 was 0.033.

(Example 13)

Aluminum was subjected to metal cutting to produce a fixture of Example 12 in which the slit 14 had the tapered structure illustrated in Fig. 15. The base 25 had a size of 50 mm × 50 mm × 70 mm (length × width × height). The slit had a width T21 of 3.8 mm at a position closest to the surface of the base, and a width T22 of 3 mm at the blocked side. The tapered structure had a length T11 of 30 mm from a lateral portion of the base 25, and the value of (T21 - T22)/T11 was 0.027.

(Example 14)

Aluminum was subjected to metal cutting to produce a fixture of Example 13 in which the slit 14 had the tapered structure and the bent structure illustrated in Fig. 17. The base 25 had a size of 50 mm × 50 mm × 70 mm (length × width × height). The slit 14 had a width T21 of 3.2 mm at a position closest to the surface of the base, and a depth T3 of 30 mm. The slit 14 was formed to extend linearly for a length of 30 mm in the insertion direction, turn to right by 90 degrees, and extend linearly for a length of 10 mm. The tapered structure had a width T22 of 3 mm at the blocked side, and a slope (T21 - T22)/T11 of 0.02.

<Evaluation of fixing condition of cryopreservation jig>

For each of the fixtures of Examples 12 to 14, a cryopreservation jig as illustrated in Fig. 32 was produced to evaluate the fixing condition of the cryopreservation jig. For the cryopreservation jig in Fig. 32, a polyethylene terephthalate resin film having a strip shape (width; 1.5 mm; length: 20 mm; thickness: 190 $\mu$m) was used as the deposition part 4, and the film was bonded to the handle 5 made of ABS resin to produce the body 2. The cap member 3 was made of ABS resin. The external shape of the cap member 3 was a quadrangular prism with a base side of 3.1 mm. The cap member 3 illustrated in Fig. 32 included no recesses.

An equilibrated mouse 8-cell stage embryo was dropped with a preservation solution (0.1 $\mu$L) to the above-described cryopreservation jig under a transmission microscope. The preservation solution had a composition containing DMSO (15 vol%), ethylene glycol (15 vol%), and sucrose (17 mass%) in Medium 199 available from Sigma-Aldrich. Then, the strip including the deposition part was inserted into the cap member under a transmission microscope, and was subsequently immersed in liquid nitrogen for freezing. After the freezing, the cap member-side of the cryopreservation jig was fixed to the slit 14 in the liquid nitrogen, and the fixing and holding state in the form illustrated in Fig. 32 was evaluated based on the following criteria. Table 3 shows the results in "Evaluation of fixing condition during thawing".

AA: The fixing was particularly securely completed, and was particularly highly evaluated.
A: The fixing was securely completed with stability.
B: The fixing was stably completed.
C: The fixing was completed but was slightly unstable.

[Table 3]

|  | Evaluation of fixing condition during thawing |
| --- | --- |
| Example 12 | B |
| Example 13 | AA |
| Example 14 | AA |

<Evaluation of working efficiency in thawing>

Each of the fixtures of Examples 3 to 14 was used to fix and hold the cryopreservation jig in the form illustrated in Fig. 33 by the same method as in "Evaluation of fixing condition of cryopreservation jig" described above. Fig. 33 is a schematic view of the body 2 and the cap member 3 being separated from each other, and the deposition part 4 being immersed in the thawing solution 24.

Subsequently, the working efficiency in separating the cap member from the body of the cryopreservation jig in the form illustrated in Fig. 33 was evaluated. The fixtures of Examples 3 to 14 allowed rapid thawing, demonstrating high working efficiency. In thawing using the fixtures of Examples 3 to 14, it was easy to confirm that the cell or tissue was in the liquid nitrogen.

These results show that use of the fixture of the present invention makes it possible to stably fix and hold the cryopreservation jig prior to transfer of the cell or tissue to the thawing solution. When the cryopreservation jig is fixed and held using the fixture of the present invention prior to transfer of the cell or tissue to the thawing solution, an operator can easily confirm that the cell or tissue deposited on the strip of the cryopreservation jig is below the liquid surface of the liquid nitrogen, and also can perform rapid thawing.

INDUSTRIAL APPLICABILITY

The present invention can be applied to cryopreservation and thawing of cells or tissues, such as cells or tissues for embryo transfer and artificial insemination of domestic animals (e.g., cattle) and other animals, and for human artificial insemination; iPS cells; ES cells; commonly used culture cells; cells or tissues, including embryos and eggs, harvested from living bodies for the purpose of examination or implantation; and cells or tissues cultured in vitro.

REFERENCE SIGNS LIST

1    cryopreservation jig
2    body
3    cap member
4    deposition part
5    handle
6    marking
7    fitting structural member
8    fitting contact portion
9    fixing portion
10    joint portion
11    cell
12    preservation solution
13    fixture
14    slit
15    irregular structure
16    slit opening

17 hollow portion
18 liquid nitrogen
19 liquid surface
20 freezing container
21 liquid surface gauge portion
22 upper limit marking
23 lower limit marking
24 thawing solution
25 base
26 insertion portion

## Claims

1. A cryopreservation jig fixture comprising:

   a base having an upper surface, side surfaces, and a bottom surface; and
   either
   a slit on the upper surface of the base having at least one end opened at the side surface of the base
   or
   an insertion portion for inserting a cryopreservation jig on the upper surface of the base and a slit having one end opened at a lateral portion of the insertion portion,
   wherein the slit has a fixing structure for fixing a cryopreservation jig, and the fixing structure is at least one of the following structures (I) to (III):

   (I) an irregular structure formed on at least one inner side surface of the slit;
   (II) a tapered structure in which a distance between inner side surfaces of the slit gradually decreases toward a back of the slit; and
   (III) a bent structure in which the slit extends linearly from a slit opening toward an inside of the base and then bends in a direction different from the linearly extending direction.

2. The cryopreservation jig fixture according to claim 1,
   wherein the slit has at least one of the tapered structure (II) and the bent structure (III) as the fixing structure.

3. The cryopreservation jig fixture according to claim 1 or 2,
   wherein the slit has the tapered structure (II) as the fixing structure, and the tapered structure satisfies the following formula (1):

$$(T21 - T22)/T11 \leq 0.03 \quad \text{formula (1)}$$

   where T21 is a width of the tapered structure at a portion closest to the slit opening, T22 is a width of the tapered structure at a portion farthest from the slit opening, and T11 is a length of the tapered structure.

4. The cryopreservation jig fixture according to any one of claims 1 to 3,
   wherein the slit has, as the fixing structure, the bent structure (III) in which the slit bends in a direction different from its linearly extending direction and the tapered structure (II) located after the bend.

5. The cryopreservation jig fixture according to any one of claims 1 to 4, further comprising a liquid surface gauge portion on the upper surface or one of the side surfaces of the base, the liquid surface gauge portion extending above the upper surface of the base.

6. A method of freezing and thawing a cell or tissue, comprising the following steps:

   a sealing step of depositing the cell or tissue with a preservation solution on a deposition part of a cryopreservation jig at least including a body including the deposition part and a cap member freely attachable to and detachable from the body, and sealing the deposition part with the cap member;
   a freezing step of cooling the sealed deposition part by a coolant to freeze the cell or tissue;

a refrigerating step of maintaining the frozen state of the cell or tissue frozen in the freezing step; and

a thawing step of thawing the cell or tissue maintained in the frozen state,

wherein prior to the thawing step, the cell or tissue sealed with the cap member is fixed to the fixing structure of the slit of the cryopreservation jig fixture according to any one of claims 1 to 5, and the cryopreservation jig is temporarily held in a state where the cell or tissue is below a liquid surface of the coolant and a joint portion between the body and the cap member is above the liquid surface of the coolant, and

in the thawing step, the body and the cap member are separated from each other, and the deposition part of the body is immersed in a thawing solution to thaw the cell or tissue.

7. The method of freezing and thawing a cell or tissue according to claim 6,
wherein the deposition part comprises a preservation solution absorber.

FIG.1

FIG.2

FIG.3

FIG.4

FIG.5

FIG.6

FIG.7

FIG.8

# FIG.9

# FIG.10

FIG.11

T6

1 5

1 4

2 5

1 3

T2

FIG.12

T5

1 5

1 4
2 5

1 3

T2

FIG.13

T2

T4

1 5

2 5

T1

1 3

1 4

1 6

FIG.14

1 5

2 5

1 3

1 4

1 6

EP 3 985 090 A1

## FIG.15

T22

2 5

$T11$
$(T1)$

1 3

1 4

1 6

T21
(T2)

## FIG.16

2 5

1 3

1 4

1 6

29

## FIG.17

## FIG.18

FIG.19

2 5

T2

1 4

1 3    2 6    1 6

FIG.20

T22

1 4

2 5

T11
(T1)

2 6

1 3

T21
(T2)

## FIG.21

## FIG.22

FIG.23

FIG.24

# FIG.25

# FIG.26

# FIG.27

# FIG.28

# FIG.29

# FIG.30

## FIG.31

# FIG.32

# FIG.33

## INTERNATIONAL SEARCH REPORT

| International application No. |
| --- |
| PCT/JP2020/021895 |

A. CLASSIFICATION OF SUBJECT MATTER
C12M  1/00(2006.01)i;  A01N  1/02(2006.01)i;  C12N  1/04(2006.01)i;  C12N 5/073(2010.01)i
FI: C12M1/00 A; A01N1/02; C12N1/04; C12N5/073

B. FIELDS SEARCHED

Minimum documentation searched (classification system followed by classification symbols)
C12M1/00; A01N1/02; C12N1/04; C12N5/073

| Documentation searched other than minimum documentation to the extent that such documents are included in the fields searched | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
| --- | --- | --- |
| Y | JP 2016-124819 A (HIROSHIMA-KEN) 11.07.2016 (2016-07-11) claims 1, 8, paragraphs [0031]-[0075] | 1-7 |
| Y | US 642697 S (GAEFVERT, Lars) 02.08.2011 (2011-08-02) entire text | 1-7 |
| Y | JP 2014-183757 A (THE KITASATO INSTITUTE) 02.10.2014 (2014-10-02) claim 1 | 7 |
| Y | JP 2015-142523 A (MITSUBISHI PAPER MILLS LIMITED) 06.08.2015 (2015-08-06) claim 1 | 7 |
| Y | WO 2015/064380 A1 (THE KITASATO INSTITUTE) 07.05.2015 (2015-05-07) claim 1 | 7 |

☒ Further documents are listed in the continuation of Box C. ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 05 August 2020 (05.08.2020) | 18 August 2020 (18.08.2020) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
|---|
| PCT/JP2020/021895 |

C (Continuation).    DOCUMENTS CONSIDERED TO BE RELEVANT

| Category* | Citation of document, with indication, where appropriate, of the relevant passages | Relevant to claim No. |
|---|---|---|
| A | ワケンビーテック株式会社・営業推進部・企画グループ編，液体窒素取り扱いの手引き（容器編）[online], 初版，20 January 2018, [retrieved on 05 August 2020], Retrieved from the Internet, <URL: https://www.wakenbtech.co.jp/wp/wp-content/uploads/2018/01/LN2-storage-tank-guide.pdf>, entire text, non-official translation (WAKENBTECH CO., LTD., Sales promotion department, Edition of the planning group, "Handling guidance for liquid nitrogen (container edition) [online]", 1st edition) | 1-7 |

Form PCT/ISA/210 (continuation of second sheet) (January 2015)

**INTERNATIONAL SEARCH REPORT**
Information on patent family members

International application No.

PCT/JP2020/021895

| Patent Documents referred in the Report | Publication Date | Patent Family | Publication Date |
|---|---|---|---|
| JP 2016-124819 A | 11 Jul 2016 | (Family: none) | |
| US 642697 S | 02 Aug. 2011 | (Family: none) | |
| JP 2014-183757 A | 02 Oct. 2014 | (Family: none) | |
| JP 2015-142523 A | 06 Aug. 2015 | WO 2015/115313 A1 | |
| WO 2015/064380 A1 | 07 May 2015 | US 2016/0235056 A1 claim 1 EP 3064567 A1 CN 105705626 A KR 10-2016-0096598 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3044323 B
- JP 2008005846 A
- JP 2017104061 A
- JP 2000189155 A
- JP 5798633 B
- WO 2019004300 A
- JP 2002315573 A
- JP 2006271395 A
- JP 2014183757 A
- JP 2015142523 A
- WO 2015064380 A
- US D642697 S
- WO 2011070973 A

**Non-patent literature cited in the description**

- **STEPONKUS et al.** *Nature,* 1990, vol. 345, 170-172
- ''Seisaibo no toketsu ni yoru shogai to hogo no kiko'' (Mechanism of damage to living cells by freezing and protection). **HIROSHI SOUZU.** Kagaku to Seibutsu (Chemistry and Biology). The Japan Society for Bioscience, Biotechnology, and Agrochemistry, 1980, vol. 18, 78-87